# EUROPEAN PATENT APPLICATION

(11) **EP 1 726 637 A1**
(43) Date of publication of application: **29.11.2006**
(21) Application number: 05719893.9
(22) Date of filing: 03.03.2005
(51) Int. Cl.: C12N 1/19, C12N 15/09, C12P 7/62

(54) **NOVEL TRANSFORMANT AND PROCESS FOR PRODUCING POLYESTER BY USING THE SAME**

(30) Priority: 04.03.2004 JP 2004061291; 05.03.2004 JP 2004062812
(71) Applicant: Kaneka Corporation, Kita-ku Osaka-shi, Osaka 530-8288 (JP)
(72) Inventor: OKUBO, Yuji, Takasago-shi, Hyogo 6760026 (JP); MATSUMOTO, Keiji, Nishinomiya-shi, Hyogo 6638023 (JP); TAKAGI, Masamichi, Fuchu-shi, Tokyo 1830051 (JP); OHTA, Akinori, Saitama-shi, Saitama 3310063 (JP)
(74) Representative: Vossius & Partner
(86) International application number: PCT/JP2005/003589
(87) International publication number: WO 2005/085415

(57) **Abstract**

The present invention provides a process for producing yeast excellent in cell productivity and gene manipulation of which is easy, being added with nutritional requirement by disrupting only a specific gene, and a transformant thereof. Moreover, the present invention also provides a process for producing a gene expression product, particularly a polyhydroxyalkanoic acid.

In the present invention, yeast in which a plurality of genes is disrupted is produced using the homologous recombination. Moreover, a transformant is obtained by introducing a plurality of enzyme genes involved with polyhydroxyalkanoic acid synthesis such as a polyhydroxyalkanoic acid synthase gene and an acetoacetyl CoA reductase gene into said gene-disrupted yeast. Furthermore, said transformant is cultured, copolyesters comprising a polyhydroxyalkanoic acid are efficiently accumulated within the cells, and a polymer is harvested from the cultured product.

## Description

### TECHNICAL FIELD

The present invention relates to a gene-disrupted strain prepared by disrupting specific chromosomal DNA in yeast by the principle of homologous recombination. Moreover, the present invention relates to the production of an industrially useful substance using said disrupted strain.

Furthermore, the present invention also relates to genes necessary for the enzymatic synthesis of copolyesters, a microorganism fermentatively synthesizing polyesters utilizing the gene, and a process for,producing polyesters using the microorganism. Furthermore, the invention also relates to a method of breeding said microorganism.

### BACKGROUND ART

Due to development of the gene recombination technologies, it became possible to mass produce industrially useful substances utilizing prokaryotic organisms and eukaryotic organisms. Among eukaryotic organisms, as yeast, those belonging to the genus Saccharomyces have been used for producing fermentative foods such as liquor through the ages, and in addition, Candida maltosa has once been used as a microbial protein-containing food or feed, thus the safety of the yeast itself has been confirmed.

Yeast grows rapidly and generally can be cultured at higher cell density than bacteria. Further, cells of yeast can be separated from the culture fluid with ease as compared with bacteria and, thus, the extraction and purification steps of products can be facilitated. Using such characteristics, yeast has been used as a production host for useful products by recombinant DNA, and the usefulness thereof has been demonstrated.

Among various yeast, unlike those belonging to the genus Saccharomyces, yeast of the genus Candida does not generate ethanol when cultivated under aerobic conditions, and is not affected by the growth inhibition caused thereby. Thus, efficient cell production and substance production by the continuous culture at high density are possible. Furthermore, asporous yeast Candida maltosa has a characteristic that it can be assimilated and bled using a straight-chain hydrocarbon having a carbon chain of C₆-C₄₀, or fats and oils such as palm oil and coconut oil as the only carbon source. Since such characteristic is practically advantageous as a place for producing or reacting useful substances by the conversion of hydrophobic chemical substances, utilization thereof for producing various compounds is expected (Wolf K. ed., Nonconventional Yeasts in Biotechnology. A Handbook, Springer-Verlag, Berlin(1996) p411-580). Moreover, using such characteristic, utilization of Candida maltosa for producing useful substances by gene recombination is also expected, and development of the gene expression system for that purpose has been intensively carried out (Japanese Kokai Publication Sho-62-74287 and Japanese Kokai Publication Sho-62-74288). Recently, it has been disclosed that Candida maltosa can be used for producing a straight-chain dicarboxylic acid by gene recombination (WO 99/04014) and as a biodegradable plastic (WO 01/88144).

As mentioned above, the host-vector system for Candida maltosa had been developed from early, and a number of auxotrophic mutants thereof have been obtained by mutagenesis treatment, but the production of a novel useful chemical substance using a recombinant has not still been industrialized. As a reason for that, there may be mentioned the fact that any auxotroph to wild strains comparable in proliferation potency and in straight-chain hydrocarbon chain utilizing capacity is not available as yet. Although CHA1 strain, which was developed by subjecting Candida maltosa to mutagenesis treatment, has been confirmed to have mutations in ADE1 gene and HIS5 gene (Kawai S. et al., Agric. Biol. Chem., 55:59-65 (1991)), the proliferation ability is inferior to that of a wild strain. This is considered to be because of mutation at another site or other sites than the targeted one.

When yeast is used as a host for the substance production by gene recombination, a selective marker (a selective signal) is used by which the introduction of the target gene can be confirmed, as in the case of using Escherichia coli, etc. In the case of yeast, as a selective marker gene providing drug resistances, a resistance-providing gene against such as cycloheximide, G418 or Hygromycin B is used. However, since there is no good drug functioning specifically to yeast, there is a phenomenon that cells not introduced with the target gene also slightly grows, or drug resistances gradually increase, and the like problems are known. Furthermore, each strain of yeast has the different extent of drug resistances, and the expression amount of a drug-resistant gene necessary for providing drug resistances in yeast also changes. Therefore, in order to provide drug resistances to each yeast, a promoter for expressing the drug-resistant gene should be appropriately selected or produced. Particularly, Candida maltosa has cycloheximide resistance from the first (Takagi et al., J. Gen. Appl. Microbiol., 31: 267-275 (1985)), and the extent of resistance to various drugs as mentioned above has not been known. Furthermore, a part of yeast species including Candida maltosa is known to have a different way of translation of codon from general pattern such as Escherichia coli and human ( Ohama T. et al., Nucleic Acid Res., 21: 40394045 (1993)). That is, there is a high possibility that a drug-resistant gene cannot be directly used.

Therefore, a selective marker having appropriate nutritional requirement is preferably used. For using a nutrition-requiring selective marker, it is required to acquire a mutant added with nutritional requirement. Conventionally, for the addition of nutritional requirement, a mutant has been acquired by a random mutation induction treatment using a mutagen such as nitrosoguanidine and ethylmethane sulfonate. However, with this mutagenesis, although the objective nutrition-requiring strain can be obtained, the possibility that the site other than the target one may be mutated cannot be denied. This becomes an obstacle in developing yeast as a host as mentioned before, and can be said as a cause for delaying the use of Candida maltosa as fields for the substance production as compared with Escherichia coli, etc.

Furthermore, as another problem of the mutant acquired by the random mutation, there is spontaneous reversion of a mutated site. In this case, since a revertant preferentially proliferates during culture, substance productivity of recombinants may decrease. Moreover, when a revertant leaks into the environment, there is a high possibility that it may survive and proliferate, and thus there is a problem in view of safety standard. Accordingly, it is not suitable to use a strain acquired by the random mutagenesis as fields for the substance production. Then, acquisition of a disrupted strain in which only genes involved with synthesis of a specific amino acid, vitamin, etc. are disrupted has been desired, and AC16 strain was produced as Candida maltosa added with nutritional requirement by disrupting only ADE1 gene (Japanese Kokai Publication 2002-209574). However, since this strain has only one species of marker, there has been a problem that gene which can be introduced was restricted.

As a method for introducing a gene into yeast, there are a method using a plasmid vector, and a method comprising incorporating the gene into a chromosomal gene.

In plasmid vectors, which are genes capable of automonous replication in cells of yeast, those in which about 1 copy occurs in each cell (YCp type), and those in which multiple copies can occur in each cell (YRp type) are now being developed for the respective yeast species. Although it is considered to be more advantageous to use the latter plasmid vector method to increase the expression amount of the target gene product, generally, there is a problem in stability of the plasmid vector in many cases, and thus it cannot be used industrially with advantage in many cases. In such cases, it is supposed to use YCp type one using a stronger promoter, which is for expressing the target gene, or increasing the number of genes introduced (copy number). Yeast is also known to be under restriction of a size of gene transferred when a gene is introduced using a plasmid vector. Although it depends on the types of a plasmid vector to be used, it is hard to say that it is industrially useful to use a vector containing a gene of too large size such as a case that a plural species of genes are to be introduced, or a plural genes of single species are to be introduced in view of the difficulty in vector production, decrease of introduction efficiency of vector into yeast, deletion of the target gene in yeast, etc. In such cases, the problems can be solved by incorporating the target gene into a chromosome. Moreover, in some cases, the target gene may be expressed at a higher level when the gene is incorporated into a chromosome. However, if there is only one species of the selective marker, when that selective marker is once used, that gene recombinant strain has no selective marker any more, thus multiple gene introductions become impossible.

From these facts, gene-disrupted yeast having a plurality of nutrition-requiring markers has been desired.

As mentioned above, as a mutant of Candida maltosa, many of gene mutants such as ADE1 gene, histidinol phosphate-aminotransferase (HIS5 gene) and orotidine-5'-phosphate decarboxylase (URA3 gene) have been acquired (Wolf K. ed., Nonconventional Yeasts in Biotechnology. A Handbook, Springer-Verlag, Berlin(1996) p411-580). However, it has been difficult to acquire Candida maltosa added with a plurality of nutritional requirement by specifically disrupting only a specific gene since said yeast showed partial diploids. Therefore, for constructing the industrially useful substance production system utilizing yeast characteristics and by gene recombination, a host having a plurality of selective markers by gene disruption has been desired. Further, not only for Candida maltosa, such a gene-disrupted strain is desired.

Furthermore, the production of industrially useful substances by Candida maltosa having a plurality of selective markers is expected utilizing a characteristic of assimilating and growing using a straight-chain hydrocarbon, fats and oils such as palm oil and coconut oil as the only carbon source.

At present, many species of microorganisms are known to accumulate polyesters such as polyhydroxyalkanoates (hereinafter referred to briefly as PHA) as the energy storage materials within cells. A representative example of the polyester is poly-3-hydroxybutyric acid (hereinafter referred to briefly as P(3HB)), which is a homopolymer of 3-hydroxybutyric acid (hereinafter referred to briefly as 3HB). It was first discovered in Bacillus megaterium in 1925 (M. Lemoigne, Ann. Inst. Pasteur, 39, 144 (1925)). P(3BH) is a thermoplastic polymer and is biodegradable in the natural environment and, thus, has recently attracted attention as an ecofriendly plastic. However, P(3HB) is high in crystallinity, and stiff and brittle material, so that the range of practical application thereof is limited. Therefore, research works have been undertaken to improve these properties.

Among them, a technology of producing a copolymer made of 3-hydroxybutyric acid (3HB) and 3-hydroxyvaleric acid (hereinafter referred to briefly as 3HV) (hereinafter such copolymer is referred to as P(3HB-co-3HV)) is disclosed (Japanese Kokai Publication Sho-57-150393 and Japanese Kokai Publication Sho-59-220192). This P(3HB-co-3HV) is rich in flexibility as compared with P(3HB), hence was expected to have a broader application range. In actuality, however, P(3HB-co-3HV) shows only slight changes in physical properties even when the molar fraction of 3HV is increased. In particular, the flexibility can not be improved to the amount required for use in films and the like. Thus, it has been used only in the field of rigid shaped articles such as shampoo bottles and disposable razor grips.

In recent years, studies have been made concerning the copolymer consisting of two components 3HB and 3-hydroxyhexanoic acid (hereinafter referred to briefly as 3HH) (hereinafter such copolyesters are referred to briefly as P(3HB-co-3HH)) and the technology of producing it (for example, Japanese Kokai Publication Hei-05-93049 and Japanese Kokai Publication Hei-07-265065). According to these patent documents, this technology of producing P(3HB-co-3HH) comprises fermentative production thereof from fatty acids, such as oleic acid, or oils or fats, such as olive oil, using Aeromonas caviae isolated from soil. Studies concerning the properties of P (3HB-co-3HH) have also been made (Y. Doi, S. Kitamura, H. Abe, Macromolecules, 28, 4822-4823 (1995)). According to this report, when A. caviae is cultured using fatty acids of not less than 12 carbon atoms as the only carbon source, P(3HB-co-3HH) with a 3HH fraction of 11 to 19 mole percent can be fermentatively produced. It has been revealed that the properties of such P(3HB-co-3HH) change from hard and brittle (which are properties of P(3HB)) gradually to soft and flexible, to an extent exceeding the flexibility of P(3HB-co-3HV), with the increase in molar fraction of 3HH. However, the above method of production is low in polymer productivity, namely the content in cells is 4 g/L and the polymer content is 30%. Therefore, methods capable of attaining higher productivity for practical use have been searched for.

A polyhydroxyalkanoic acid synthase (hereinafter referred to briefly as PHA synthase) gene has been cloned from Aeromonas caviae, which is a producer strain of P(3HB-co-3HH) (Japanese Kokai Publication Hei-10-108682; T. Fukui, Y. Doi, J. Bacteriol., vol. 179, No. 15, 4821-4830 (1997)). This gene was introduced into Ralstonia eutropha (formerly Alcaligenes eutrophus), and cultivation was carried out using the resulting transformant and a vegetable oil as the carbon source, whereby a content in cells of 4 g/L and a polymer content of 80% were attained (T. Fukui et al., Appl. Microbiol. Biotechnol., 49, 333 (1998)). A method of producing P(3HB-co-3HH) using bacteria, such as Escherichia coli, or a plant as a host has also been disclosed, without describing any productivity data, however (WO 00/43523, for example).

The above polyester P(3HB-co-3HH) can be given a wide range of physical properties, from properties of rigid polymers to properties of flexible polymers, by changing the molar fraction of 3HH and therefore can be expected to be applicable in a wide range, from television boxes and the like, for which rigidity is required, to yarns, films and the like, for which flexibility is required. However, the production methods mentioned above are still poor in the productivity of P(3HB-co-3HH). There is no other way but to say that they are still unsatisfactory as practical production methods of P(3HB-co-3HH).

Some studies of the production of biodegradable polyesters using yeast high in cell productivity as a host have been reported. Leaf et al. have confirmed the accumulation of P(3HB) by introducing the PHA synthase gene of Ralstonia eutropha into Saccharomyces cerevisiae, a species of yeast, to produce a transformant, and by culturing the transformant using glucose as the carbon source (Microbiology, vol. 142, pp. 1169-1180 (1996)). However, polymer content achieved in this study resulted in as low as 0.5% and the polymer was stiff and brittle P(3HB).

Other studies also have been made on the production of copolymers containing a monomer unit having 5 or more carbon atoms, by expressing, in yeast Saccharomyces cerevisiae, a PHA synthase gene derived from Pseudomonas aeruginosa in the presence of fatty acids as the carbon source. But the polymer content achieved also resulted in as low as 0.5% in this case (Poirier Y. et al., Appl. Microbiol. Biotechnol. 67, 5254-5260 (2001)).

According to another investigation, by introducing β ketothiolase synthesizing 3-hydroxybutyryl-CoA by dimerizing acetyl-CoA and an NADPH-dependent reductase gene together with a PHA synthase gene, the accumulation of polymer in 6.7% per cell weight has been confirmed (Breuer U. et al., Macromol. Biossci., 2, pp 380-386 (2002)). However, these polymers were P(3HB) having stiff and brittle properties.

Furthermore, other studies have been made on the production of polyester using oleic acid as the carbon source, by expressing and targeting, into peroxisomes of yeast Pichia Pastoris, a PHA synthase gene derived from the genus Pseudomonas. These studies showed that 1% weight of polymer was accumulated per dried cell unit ( Poirier Y. et al., FEMS Microbiology Lett., vol. 207, pp.97-102 (2002)). But such low accumulation content is quite insufficient for the industrial production.

Yeast is known to grow fast and be high in cell productivity. Among them, yeasts belonging to the genus Candida attracted attention as single cell proteins in the past and, since then, studies have been made on the production of cells thereof for use as feeds using normal-paraffins as carbon sources. Further, in recent years, host-vector systems for the genus Candida have been developed, and the production of substances using the recombinant DNA technology has been reported (Kagaku to Seibutsu (Chemistry and Living organisms), vol. 38, No. 9, 614 (2000)). When Candida utilis is used as a host, the α-amylase productivity is as high as about 12.3 g/L. Microorganisms of the genus Candida having such high substance productivity are expected to serve as hosts for polymer production. Furthermore, cells thereof can be separated from the culture fluid with ease as compared with bacteria and, thus, the polymer extraction and purification steps can be facilitated.

Thus, a method of producing P(3HB-co-3HH) having good physical properties using yeast belonging to the genus Candida, and the like, has been developed, but further improvement has been requested for polymer productivity (WO 01/43523). As one method for increasing polymer productivity per cell, a method of increasing the expression amount of enzyme genes involved with PHA synthesis in cells was supposed.

In vectors, which are genes capable of automonous replication in cells of yeast, those in which about 1 copy occurs in each cell (YCp type), and those in which multiple copies can occur in each cell (YRp type) are now being developed for the respective yeast species. Also in Candida maltosa, the causal region of high-efficient transformation (transformation ability: hereinafter referred to briefly as TRA) containing the sequence involved with automonous replication (autonomously replicating sequence: hereinafter referred to briefly as ARS) and centromere sequence (hereinafter referred to briefly as CEN) was found by M. Kawamura et al. (M. Kawamura, et al., Gene, vol.24, 157,(1983)), and a low-copy vector with high stability having TRA whole region and a vector with high copy numbers removed with CEN region which can be expected to have high transgene expression have been developed (M. Ohkuma et al., Mol. Gen. Genet., vol.249, 447,(1995)). However, high-copy vectors such as Candida maltosa have problems in stability, and cannot be industrially used with advantage. Accordingly, it has been difficult to improve the polymer productivity by increasing the expression amount of enzyme genes involved with PHA synthesis within cells using a vector with high copy numbers.

Moreover, as a method of increasing the expression amount of the enzyme gene involved with PHA synthesis within a cell, a method can be supposed which comprises making a promoter, which expresses said gene, stronger. From Candida maltosa, various promoters have been cloned. A promoter of phosphoglycerate kinase (hereinafter referred to briefly as PGK), which is known as an enzyme of glycolysis system, induces strong gene expression in the presence of glucose. Furthermore, GAL promoter having strong gene expression-inducing activity in the presence of galactose has also been cloned (S. M. Park et al., Yeast, vol.13, 21 (1997)). However, these promoters hardly function when using, as an example, fats and oils and fatty acids or normal alkane (n-alkane) suitable for producing P(3HB-co-3HH) as the carbon source. Furthermore, since GAL promoter is induced only when galactose is used as the carbon source, it can be said it is not suitable for the industrial production from the viewpoint that expensive galactose must be used.

Candida maltosa produces enzymes of n-alkane oxidization system at high levels in the presence of alkane. Particularly, a gene coding for cytochrome P450 causing initial oxidization (hereinafter referred to briefly as ALK) is strongly induced (M. Ohokuma, et al., DNA and Cell Biology, vol.14, 163 (1995)). However, even with these promoters, the activity is still lower as compared with PGK or GAL promoter. Furthermore, promoters such as actin synthase 1 gene constitutively expressed (hereinafter referred to briefly as ACT1) cannot be said to have sufficient strength in activity. As an example, when fats and oils, fatty acids or n-alkane suitable for producing P(3HB-co-3HH) is used as the carbon source, at present, a promoter stronger than ARR (alkane responsible region) promoter having improved promoter activity by adding multiple ARR sequences to the upstream of ALK2 promoter (Kogure et al., Summaries of Japan Agricultural Chemical Convention Lecture in 2002, p 191) has not been developed yet. Accordingly, it is not realistic to use a strong promoter as a method of increasing the expression amount of enzyme genes involved with PHA synthesis within cells.

Asides from this, a method of introducing many expression units of the enzyme gene involved with PHA synthesis to a vector can be supposed, but in the case of introducing a gene using a vector, it is known that yeast is also under restriction of the size of a gene transferred. Thus, it is hard to say it is industrially realistic to use a vector containing a gene of too large size in view of the difficulty in vector construction, transformation efficiency into yeast, stability in yeast, and the like viewpoint.

Moreover, the method of increasing the target enzyme activity by amplifying a gene has been reported ( K. Kondo, et al., Nat. Biotechnol., vol.15, pp453-457 (1997)). This method comprises ligating a cycloheximide resistance gene and the target gene, introducing thereof into yeast having cycloheximide sensitivity, and acquiring a strain resistant to higher cycloheximide concentration to obtain a strain expressing the target gene at higher levels. However, Candida maltosa is known to have cycloheximide resistance and it is difficult to increase the expression amount of the enzyme gene involved with PHA synthesis within cells by such gene amplification using a cycloheximide resistance gene.

Then, development of a process for producing biodegradabile polyesters at high levels has been desired which avoids the above problems, and increases the expression amount of the enzyme gene involved with PHA synthesis within cells in Candida maltosa.

Furthermore, it is generally known that the molecular weight of polyesters largely affects physical properties or workability. In the PHA production within microorganisms, when the molecule numbers of enzyme per cell is excessively increased, the substrate concentration restricts the enzyme reaction, and thus the molecular weight of the polymer produced is decreased (Sim S.J. et al., Nature Biotechnology, vol.15, pp 63-67 (1997); and Gerngross T.U., Martin D.P., Proc. Natl. Acad. Sci. USA, vol.92, pp6279-6283 (1995)). Therefore, the development of a method for controlling the molecular weight of polyesters produced within cells have been desired. Moreover, when the produced polyester is a copolymer, it is also known that the monomer composition also largely affects physical properties or workability. Therefore, the development of a method for controlling the monomer composition of copolyesters has also been desired.

In addition, for breeding a strain producing PHA at high levels, it is necessary to increase the expression amount of the enzyme gene involved with PHA synthesis within cells, and in some cases, while taking the productivity and physical properties of PHA produced by a transformed strain introduced with said gene group into consideration, further introduction of said gene group is required. Generally, for acquiring a transformed strain, markers for drug resistances and nutritional requirements, etc. are used. Therefore, markers are required in number of species correspondent to the number of gene introduction, but Candida maltosa having a plurality of gene markers which has so far been developed is greatly inferior in the growth rate as compared with that of wild strains, and thus the use thereof as a PHA-producing strain has been difficult (Kawai S. et al., Agric. Biol. Chem., 55:59-65 (1991)). Moreover, Candida maltosa improved with the growth rate having one species of gene marker has also been developed (Japanese Kokai Publication 2002-209574). However, it was considered difficult to further add a plurality of gene markers while retaining the growth rate equivalent to that of wild strains to the strain since said yeast has a genome of diploid.

### SUMMARY OF THE INVENION

In view of the above state of the art, the present invention provides an industrially useful novel host into which a number of various genes can be introduced and which is capable of producing a useful substance at a high efficiency by constructing a multiple nutrition-requiring gene-disrupted strain in yeast, particularly of the genus Candida.

The present invention also provides, in view of the above state of the art, a yeast transformant transformed by a plurality of gene expression cassettes of a gene involved with PHA synthesis, and a process for producing polyesters such as P(3HB-co-3HH) having biodegradability and good physical properties which comprises culturing the transformant obtained in the above manner. Furthermore, the present invention also provides a method of breeding said microorganism.

The present inventors have made intensive investigations to solve the above-mentioned subjects, and as a result, they produced an ADE1 gene-, HIS5 gene- and URA3 gene-disrupted strain by making full use of gene recombination technologies using fragments of DNA coding for phosphoribosyl aminoimidazole-succinocarboxamide synthase of yeast (EC6.3.2.6) (ADE1 gene), DNA coding for histidinol-phosphate-aminotransferase (EC2.6.1.9) (HIS5 gene), and DNA coding for orotidine-5'-phosphate decarboxylase (EC4.1.1.23) (URA3 gene) by the principle of homologous recombination with chromosomal DNA, and succeeded in acquiring an adenine-, histidine- and uracil-requiring gene-disrupted yeast. Then, they completed the present invention in comparing the proliferation ability of said gene-disrupted yeast with that of AC16, an ADE1 gene-disrupted strain of Candida maltosa, and showing that the ability was equivalent to the AC16 strain, which has been verified to have superior proliferation ability to CHA1, an ADE1 gene mutant prepared by subjecting Candida maltosa to mutagenesis treatment. The present inventors further have made intensive investigations to solve the above subjects, and as a result, they produced a transformant in which a plurality of polyhydroxyalkanoic acid synthases genes (hereinafter referred to briefly as phaC) and acetoacetyl CoA reductase genes (EC1.1.1.36) (hereinafter referred to briefly as phbB) are introduced into a gene-disrupted strain of Candida maltosa by making full use of gene recombination technologies. Thereby, they succeeded in efficiently producing a copolyester useful as a biodegradable polyester comprising two components 3-hydroxybutyrate (hereinafter referred to briefly as 3HB) and 3-hydroxyhexanate (hereinafter referred to briefly as 3HH) (hereinafter such copolyesters are referred to briefly as P(3HB-co-3HH)).

That is, the first aspect of the present invention relates to
a yeast wherein URA3 gene of chromosomal DNA is disrupted by the homologous recombination with a URA3 DNA fragment; and
a yeast wherein HIS5 gene of chromosomal DNA is disrupted by the homologous recombination with an HIS5 DNA fragment.

At the same time, the present invention relates to
a yeast wherein both ADE1 gene and URA3 gene are disrupted;
a yeast wherein both ADE1 gene and HIS5 gene are disrupted;
a yeast wherein both URA3 gene and HIS5 gene are disrupted; and
a yeast wherein ADE1 gene, URA3 gene and HIS5 gene are disrupted.

Moreover, the present invention also relates to
a transformant of the above gene-disrupted yeast
which is transformed with a DNA sequence containing an isogene or heterogene.

Furthermore, the present invention provides a process for producing an industrially useful substance by acquiring a transformant prepared by introducing a plurality of heterogene expression systems into said gene-disrupted strain. Specifically, as an example, they used the transformant prepared by introducing preferably a plurality of polyhydroxyalkanoic acid synthase genes (hereinafter referred to briefly as phaC), which is an enzyme synthesizing a copolyester comprising two components 3-hydroxybutyrate (hereinafter referred to briefly as 3HB) useful as a biodegradable polyester and 3-hydroxyhexanate (hereinafter referred to briefly as 3HH) (hereinafter such copolyesters are referred to briefly as P(3HB-co-3HH)) and acetoacetyl CoA reductase genes (EC1.1.1.36) (hereinafter referred to briefly as phbB) into said gene-disrupted Candida maltosa strain according to the invention, and succeeded in efficiently producing P(3HB-co-3HH). That is, the present invention relates to a process for producing a gene expression product (particularly polyester) using a gene-disrupted yeast, a process for producing a polyester using a transformant introduced with a plurality of genes involved with polyester biosynthesis at the same time, and a process for producing a polyester which comprises harvesting a polyester from a cultured product obtainable by culturing the above transformant.

Moreover, the present invention relates to a process for producing a polyester which comprises controlling the physical properties of the produced polyester. Furthermore, the present invention also relates to an efficient recovery method of a selective marker used for gene introduction.

That is, the second aspect of the present invention relates to
a yeast transformant
which is introduced with a polyhydroxyalkanoic acid synthase gene and an acetoacetyl CoA reductase gene, and both or either of these genes being introduced in 2 or more copies.

The present invention also relates to
a process for producing a polyester using said yeast transformant,
which comprises harvesting a polyester from a cultured product obtainable by culturing said yeast transformant.

The present invention further relates to
a method for controlling the molecular weight of a polyester in producing a polyester using said yeast transformant,
which comprises controlling the number of acetoacetyl CoA reductase gene in the yeast transformant.

The present invention further relates to
a method for controlling a hydroxyalkanoic acid composition of a polyester in producing a polyester using said yeast transformant,
which comprises controlling the number of a polyhydroxyalkanoic acid synthase gene in the yeast transformant.

The present invention further relates to
a method for recovering a selective marker
which comprises carrying out the intramolecular homologous recombination in Candida maltosa having a selective marker gene to remove said selective marker gene.

### DETAILED DESCRIPTION OF THE INVENTION

Hereinafter, the present invention is described in detail.

First, as the gene-disrupted yeast of the first aspect of the invention, there may be mentioned the following.
A uracil-requiring gene-disrupted yeast
wherein URA3 gene of chromosomal DNA is disrupted by the homologous recombination with a URA3 DNA fragment;
A histidine-requiring gene-disrupted yeast
wherein HIS5 gene of chromosomal DNA is disrupted by the homologous recombination with an HIS5 DNA fragment;
An adenine- and uracil-requiring gene-disrupted yeast
wherein ADE1 gene and URA3 gene of chromosomal DNA are disrupted by the homologous recombination with an ADE1 DNA fragment and URA3 DNA fragment;
An adenine- and histidine-requiring gene-disrupted yeast
wherein ADE1 gene and HIS5 gene of chromosomal DNA are disrupted by the homologous recombination with an ADE1 DNA fragment and HIS5 DNA fragment;
A uracil- and histidine-requiring gene-disrupted yeast
wherein URA3 gene and HIS5 gene of chromosomal DNA are disrupted by the homologous recombination with a URA3 DNA fragment and HIS5 DNA fragment;
An adenine-, uracil- and histidine-requiring gene-disrupted yeast
wherein ADE1 gene, URA3 gene and HIS5 gene of chromosomal DNA are disrupted by the homologous recombination with an ADE1 DNA fragment, URA3 DNA fragment and HIS5 DNA fragment.

There is no particular restriction for the yeast to which disruption of ADE1 gene, URA3 gene, and HIS5 gene is carried out by the homologous recombination, and yeasts deposited with the deposition organizations of strains (for example, IFO, ATCC, etc.) can be used. Preferably, in view of assimilation ability of hydrophobic substances, etc. such as a straight-chain hydrocarbon, there may be mentioned yeasts belonging to the genus Candida, the genus Clavispora, the genus Cryptococcus, the genus Debaryomyces, the genus Lodderomyces, the genus Metschnikowia, the genus Pichia, the genus Rhodosporidium, the genus Rhodotorula, the genus Sporidiobolus, the genus Stephanoascus, the genus Yarrowia, and the like can be used.

Among these yeasts, those belonging to the genus Candida are more preferred from the viewpoints that the analysis of chromosomal gene sequence is advanced, host-vector system is also applicable, and the assimilation ability of a straight-chain hydrocarbon, fats and oils, etc. is high.

Among yeasts belonging to the genus Candida, particularly in view of having high assimilation ability of a straight-chain hydrocarbon, fats and oils, etc., ones of the albicans species, ancudensis species, atmosphaerica species, azyma species, bertae species, blankii species, butyri species, conglobata species, dendronema species, ergastensis species, fluviatilis species, friedrichii species, gropengiesseri species, haemulonii species, incommunis species, insectrum species, laureliae species, maltosa species, melibiosica species, membranifaciens species, mesenterica species, natalensis species, oregonensis species, palmioleophila species, parapsilosis species, pseudointermedia species, quercitrusa species, rhagii species, rugosa species, saitoana species, sake species, schatavii species, sequanensis species, shehatae species, sorbophila species, tropicalis species, valdiviana species, viswanathii species, etc. are preferably used.

Among these species, particularly ones of the maltosa species are preferred in view of the proliferation rate when a straight-chain hydrocarbon is used as the carbon source, and having high safety differ from the albicans species, etc.

Moreover, as for the production and use of the ADE1, URA3 and HIS5 gene-disrupted strain of the invention, as a preferable example of yeast, Candida maltosa can be used.

The Candida maltosa AC16 strain, which is ADE1 gene-disrupted yeast to be used in the present invention, has been internationally deposited on the Budapest Treaty with the National Institute of Advanced Industrial Science and Technology, Central 6, 1-1-1 Higashi, Tsukuba, Ibaraki, Japan, on November 15, 2000 under the accession number FERM BP-7366.

Moreover, each of the gene-disrupted yeast obtained in the present invention as mentioned below, namely
Candida maltosa U-35 strain, which is a URA3 gene-disrupted yeast (accession number FERM P-19435, date of deposit July 18, Heisei 15),
Candida maltosa CH-I strain, which is an HIS5 gene-disrupted yeast (accession number FERM P-19434, date of deposit July 18, Heisei 15),
Candida maltosa UA-354 strain, which is an ADE1 and URA3 gene-disrupted yeast (accession number FERM P-19436, date of deposit July 18, Heisei 15),
Candida maltosa AH-I5 strain, which is an ADE1 and HIS5 gene-disrupted yeast (accession number FERM P-19433, date of deposit July 18, Heisei 15),
Candida maltosa HU-591 strain, which is an HIS5 and URA3 gene-disrupted yeast (accession number FERM P-19545, date of deposit October 1, Heisei 15), and
Candida maltosa AHU-71 strain, which is an ADE1, HIS5, and URA3 gene-disrupted yeast (accession number FERM BP-10205, date of deposit August 15, Heisei 15)
has been deposited with the National Institute of Advanced Industrial Science and Technology, Central 6, 1-1-1 Higashi, Tsukuba, Ibaraki, Japan.

Herein, the homologous recombination refers to the recombination occurred in portions in which the base sequences of DNA has the similar or the same DNA sequences (homologous sequence).

Gene disruption refers to, for preventing functions of a certain gene from exhibiting, mutate the base sequence of said gene, insert another DNA, or delete certain part of the gene. As a result of gene disruption, said gene cannot be transcribed to mRNA, and the structural gene cannot be translated, or since the transcribed mRNA is incomplete, the amino acid sequence of the translated structural protein causes mutation or deletion, thereby the original functions cannot be exhibited.

ADE1 gene represents a gene fragment comprising 5'untranslated region containing a promoter region, a region coding for phosphoribosyl aminoimidazole-succinocarboxamide synthase (EC6.3.2.6), and 3' untranslated region containing a terminator region. The base sequence of ADE1 gene of Candida maltosa is disclosed on GenBank: D00855.

URA3 gene represents a gene fragment comprising 5' untranslated region containing a promoter region, a region coding for orotidine-5'-phosphate decarboxylase (EC4.1.1.23), and 3'untranslated region containing a terminator region. The base sequence of URA3 gene of Candida maltosa is disclosed on GenBank: D12720.

HIS5 gene represents a gene fragment comprising 5' untranslated region containing a promoter region, a region coding for histidinol-phosphate-amino transferase (EC2.6.1.9), and 3' untranslated region containing a terminator region. The base sequence of HIS5 gene of Candida maltosa is disclosed on GenBank: X17310.

An ADE1 DNA fragment represents DNA which can cause the homologous recombination with ADE1 gene on a chromosome within microbial cells, and thereby can disrupt ADE1 gene.

A URA3 DNA fragment represents DNA which can cause the homologous gene recombination with URA3 gene on a chromosome within microbial cells, and thereby can disrupt URA3 gene.

An HIS5 DNA fragment represents DNA which can cause the homologous gene recombination with HIS5 gene on a chromosome within microbial cells, and thereby can disrupt HIS5 gene.

Next, the transformant of the invention is one obtainable by transforming the above gene-disrupted yeast with a DNA sequence containing an isogene or heterogene.

The isogene refers to a gene occurring on a chromosome of the host yeast, or a part of DNA thereof. The heterogene refers to a gene which is not originally occurring on a chromosome of the host yeast, or a part of DNA thereof.

Moreover, a gene expression cassette can also be used. The gene expression cassette is constituted from a transcription promoter DNA sequence, DNA coding for a gene aiming at expression, and DNA containing a terminator terminating the transcription. And there are ones having the form of a circle plasmid and functioning outside a chromosome, and ones incorporated into chromosomal DNA.

Next, the process for producing the gene expression product according to the invention comprises harvesting an expression product of an isogene or heterogene from a cultured product obtainable by culturing the above transformant. Said gene expression product is particularly preferably a polyester.

As regarding the gene expression product, when a substance expressed by the gene (gene expression product) is a desired protein or enzyme, the protein or enzyme itself is the gene expression product. Moreover, when the gene expression product is various enzymes or coenzymes, a substance produced by the expression of the catalytic activity of said enzymes in the host yeast, which is directly different from a gene expression product, is also referred to as the gene expression product.

A PHA stands for a polyhydroxyalkanoate, and represents a biodegradable polyester producible by copolymerizing 3-hydroxyalkanoic acids.

A phaC represents a polyhydroxyalkanoic acid synthase gene synthesizing a biodegradable polyester producible by copolymerizing 3-hydroxyalkanoic acids.

A phbB represents an acetoacetyl CoA reductase gene synthesizing 3-hydroxybutyryl-CoA by reducing acetoacetyl CoA.

In the following, the process for producing an ADE1, URA3 and HIS5 gene-disrupted strain, and a process for producing a polyester using said disrupted strain are specifically described.

### (1) Process for producing a URA3 gene-disrupted strain, and a URA3 and ADE1 gene-disrupted strain

As for the production of a URA3 gene-disrupted strain, any methods can be used provided that a disrupted strain in which a URA3 enzyme is not expressed. Various methods have been reported as a gene disruption method, but in view of capable of disrupting only a specific gene, gene disruption by the homologous recombination is preferred (Methods in Molecular Biology, 47, edited by Nickoloff J.A.: 291-302 (1995), Humana Press Inc., Totowa, NJ). Among the homologous recombination, gene substitution disruption is preferable since a disrupted strain which is not spontaneously reverted can be acquired, and as a result a strain high in safety in handling the recombinant can be obtained.

As the URA3 DNA fragment, generally, a DNA fragment in which partial DNA inside the gene is removed and the remained both terminal portions are ligated again is used.

The partial DNA to be removed is a portion in which URA3 gene cannot exhibit enzyme activity by the removal, and DNA having a length that URA3 enzyme activity is not recovered by spontaneous reversion. The chain length of such partial DNA is not particularly restricted, but preferably 50 bases or more, and more preferably 100 bases or more. Moreover, into the removed DNA site, any length of DNA may be inserted.

These DNA fragments can be prepared by, for example, PCR method (polymerase chain reaction method), cutting with a restriction enzyme from a vector and re-ligation, or the like technology. The homology region length of both terminals of the URA3 DNA fragment is sufficiently 10 bases or more, preferably 200 bases or more, and more preferably 300 bases or more.

Moreover, the homology of the respective both terminals is preferably 90% or more, more preferably 95% or more.

URA3 gene has been predicted to occur in two or more in the chromosome of Candida maltosa. A selective marker is not necessary when there is a technology for detecting disruption of the target gene, but in the case of disrupting a gene generally occurring in a chromosome in two or more, etc., it is necessary to detect homologous recombination of the disruption object gene in a yeast chromosome using a selective marker as an index. Using a plurality of selective markers, or after disrupting the first gene and then removing or disrupting the used selective marker, an operation for disrupting the second and later genes is necessary.

Accordingly, a method comprising inserting a gene which can be a selective marker of ADE1, etc. to the removed gene site in a URA3 DNA fragment can be used. The length of the selective marker to be inserted is not particularly restricted and it is sufficient that a promoter region, structural gene region, and terminator region which can substantially function in yeast are contained. It is also allowable that the gene marker is derived from a living organism different from the target yeast.

Furthermore, by inserting an hisG gene fragment (a fragment of Salmonella ATP phosphoribosyl transferase gene; plasmid pNKY1009 containing this gene fragment is available from ATCC (ATCC: 87624)) to both terminals of the selective marker gene, the marker gene inserted can be removed after gene disruption by intramolecular homologous recombination (Alani et al., Genetics, 116: 541-545 (1987)). The gene fragment used for removing a selective marker gene in such manner is not particularly restricted, and to the upstream and downstream of the selective marker, a homologous fragment of any genes may be arranged. Therefore, it is also possible to use the sequence included in the selective marker. In the invention, by coupling a gene fragment of the 5' terminal portion of ADE1 gene to be used as a marker to the 3' terminal of ADE1 gene, it becomes possible to remove the marker gene by the intramolecular homologous recombination quite efficiently. The gene fragment to be used for the intramolecular homologous recombination of the marker is not particularly restricted, and a gene fragment in which the marker gene does not substantially function may be used. It is also possible to use a gene fragment of the 3' terminal portion.

In the present invention, three species of DNA for URA3 disruption were used.

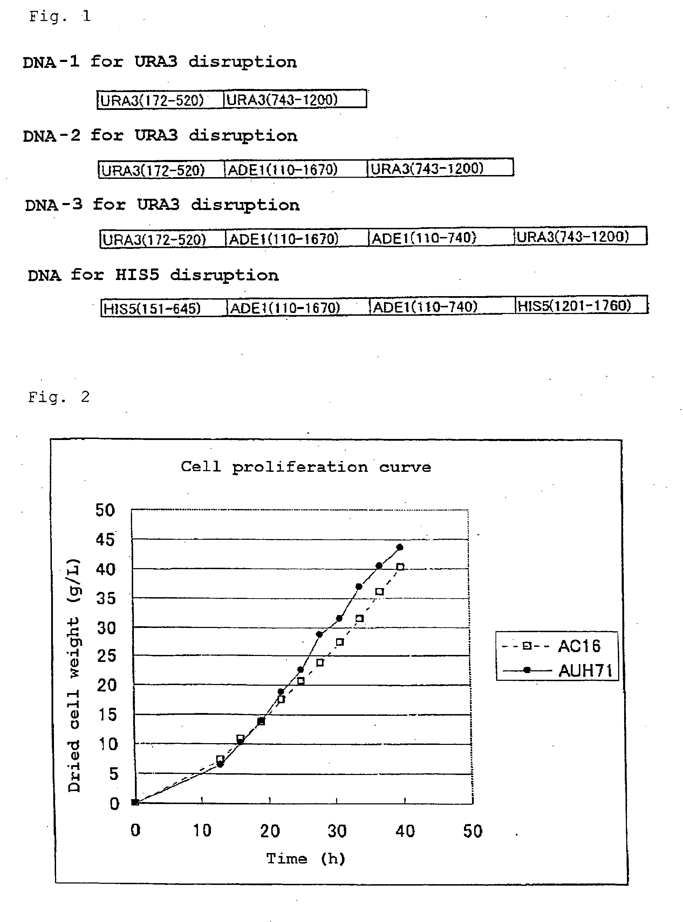

DNA-1 for URA3 disruption is DNA in which a DNA fragment coding for a URA3 enzyme of about 220 bp is removed, and the 5' side DNA fragment of about 350 bp and 3' side DNA fragment of about 460 bp are ligated (Fig. 1). The removed portion corresponds to 30% of a URA3 enzyme protein. Moreover, the homology of the 5' side and 3' side DNA fragments and the original URA3 gene are both 100%.

DNA-2 for URA3 disruption was prepared by inserting ADE1 gene derived from Candida maltosa in lieu of a URA3 DNA fragment removed in DNA-1 for URA3 disruption (Fig. 1).

In DNA-3 for URA3 disruption, the 5' terminal portion of ADE1 gene of about 630 bp is used as a sequence for causing the intramolecular homologous recombination and recovering adenine requirement, which is connected to the downstream of ADE1 gene (Fig. 1).

The DNA fragment used in the invention can be constructed on a general vector.

In the practice of the invention, pUC-Nx was used. pUC-Nx is a vector prepared by substituting DNA between EcoRI and HindIII sites of pUC19 (Molecular cloning, edited by Sambrook et al.: A Laboratory Manual, Second Edition 1.13, Cold Spring Harbor Laboratory Press (1989)) with DNA shown under SEQ ID No:1, and constructing a novel restriction enzyme site.

From pUC119-URA3 (Ohkuma M. et al., Curr. Genet., 23: 205-210 (1993)), the 5' side and 3' side of URA3 gene are separately amplified using PCR, sequentially ligated to pUC-Nx, and a vector containing DNA-1 for URA3 disruption was produced.

Next, into the same vector, ADE1 gene amplified by the PCR method was inserted, and a vector containing DNA-2 for URA3 disruption was produced in such manner.

Furthermore, the 5' terminal portion sequence of about 630 bp of ADE1 amplified by PCR was inserted into the 3' terminal of ADE1 gene in this vector, and a vector containing DNA-3 for URA3 disruption capable of removing a marker gene was produced.

The vectors containing DNA for disruption are introduced into appropriate Escherichia coli, for example JM109 or DH5α, and said Escherichia coli was cultured, then highly pure plasmids are prepared in a large amount with a cesium chloride ultracentrifugal method (Molecular cloning, edited by Sambrook et al.: A Laboratory Manual, Second Edition 1.42-1.47, Cold Spring Harbor Laboratory Press (1989)). Moreover, it is also possible to use an alkali method, etc. (Brinbioim H.C., et al., Nucleic Acids Res.7: 1513-1523 (1979)). It is further sufficiently possible to use a commercially available plasmid purification kit, etc. This vector can be directly used for gene disruption, but it is desirable to cut a portion having homology containing a URA3 region with an appropriate restriction enzyme from the purified vector, and to use the resultant as DNA for disruption. It is also possible to carry out amplification using the PCR method. In the invention, restriction enzymes SphI and SwaI were used for cutting, and a DNA fragment was introduced into cells without purification, then URA3 gene could be disrupted by the homologous recombination.

As the method for transforming Candida maltosa, a protoplast method, lithium acetate method (Takagi M. et al., J Bacteriol, 167: 551-5 (1986)), and electric pulse method (Kasuske A. et al., Yeast 8: 691-697(1992)) are known. In the practice of the invention, the electric pulse method was used. For generating electric pulse, commercially available apparatus can be used. In the invention, ELECTRO CELL MANIPULATOR 600 manufactured by BTX (San Diego, CA USA) was used. As a cuvette, BM6200 (2 mm gap blue cap) manufactured by BIO MEDICAL CORPORATION CO. LTD (Tokyo Japan) was used.

From the AC16 strain, competent cells are prepared, subjected to electric pulse together with DNA-2 for URA3 disruption, cultured in a medium not containing adenine, and a disrupted strain in which ADE1 gene is inserted into the target URA3 gene is screened from the appeared colony.

Screening of the target gene-disrupted strain can be easily carried out from the obtained colony by the PCR method or genomic southern hybridization method (Molecular cloning, edited by Sambrook et al.: A Laboratory Manual, Second Edition 9.31-9.57, Cold Spring Harbor Laboratory Press (1989)). In the PCR method, when the both terminals of URA3 gene are used as primers, in agarose gel electrophoresis, a normal size of DNA band is detected in a wild strain, but in a disrupted strain, a band which is larger for a size of an inserted gene is also detected. In the invention, DNA bands of about 1 kbp and 2kbp were detected. However, in the case of substitution with or incorporation of chromosomal DNA such as in gene disruption, the possibility that a gene may be inserted into the site other than targeted, for example unknown site having high homology should be assumed. In such cases, confirmation may be impossible by the PCR method in some cases. In this case, confirmation becomes possible by carrying out the genomic southern hybridization method, or the PCR method using a gene sequence occurring in the outside part from the site used for the homologous recombination of the disruption object gene.

URA3 gene was predicted to occur in two or more in the chromosome of Candida maltosa. Actually, a strain obtained by transforming DNA-2 for URA3 disruption showed no uracil requirement, and thus uracil requirement cannot be given if the second URA3 gene is not disrupted. Disruption of the second URA3 gene can be attained, by using a technology of disrupting the integrated ADE1 gene, and then disrupting again as the first URA3 gene disruption, or by carrying out transformation with DNA-1 for URA3 disruption, etc. and then selecting a colony growing under the coexistence of uridine or uracil and 5-FOA (5-fluoro-orotic-acid). In the invention, the former technology was used. That is, to a strain which becomes to have no adenine requirement by transforming DNA-2 for URA3 disruption, DNA-1 for URA3 disruption was electrically introduced to disrupt the first URA3 gene again, the resultant was spread on a minimum medium containing adenine, and then adenine-requiring strain can be obtained by selecting the appeared red colony. It is also possible to use an ADE1 DNA fragment (Japanese Kokai Publication 2002-209574). Thereafter, an operation for disrupting the second URA3 gene is carried out.

In this occasion, it is preferable to use a concentration process for efficiently carrying out screening. For example, a method called nystatin concentration (Snow R. Nature 211: 206-207(1966)) can be used. This method has been developed for efficiently selecting a mutant obtainable from yeast by random mutation, but can be applied to a gene-disrupted strain. For example, after gene introduction, the cultured cells are sown on YM medium, etc. and cultured. The cells are washed, cultured in a minimum medium containing no nitrogen source, and then cultured in a minimum medium containing a nitrogen source for a short time. To this culture fluid, nystatin is directly added and the cells are cultured at 30°c for 1 hour aerobically, thereby wild strains can be preferentially killed. This cell solution is smeared on an appropriate agar medium plate containing adenine and cultured at 30°c for about two days, then a red colony can be obtained.

The obtained adenine-requiring strain can be confirmed by the PCR method. When both terminals of URA3 gene are used as primers, in agarose gel electrophoresis, a normal size of DNA band is detected in the original strain, but in an ADE1-disrupted strain, a band shorter for the length of deleted portion is also detected.

Next, the second URA3 gene is disrupted by repeating the above method to this strain in which one URA3 gene is disrupted and adenine requirement is recovered, then a strain becomes to have uracil requirement can be obtained. Thereafter, by disrupting ADE1 gene again, a double nutrition-requiring strain can be obtained. In the invention, for the disruption of the second URA3 gene, URA3 gene for URA3 disruption containing ADE1 gene and capable of removing a marker gene by the intramolecular homologous recombination was used. This gene for disruption was electrically introduced into this strain, and a colony is caused to form in a selective medium containing uridine or uracil. By replicating the obtained colony to a medium containing neither uridine nor uracil, a uracil-requiring strain is selected. The chromosomal gene of the obtained requiring strain is analyzed by the PCR method, etc., and the strain in which a gene equivalent to normal URA3 is not amplified, and only a gene containing an inserted gene and a gene containing deletion are amplified are selected. At this stage, a URA3-disrupted strain is completed.

Then, the inserted ADE1 gene is removed. As this method, by applying the nystatin concentration method, it is possible to produce a strain in which ADE1 gene is spontaneously deleted by the intramolecular homologous recombination with ease. According to the preferable aspect of the invention, after the cultured cells are sown on YM medium, etc. and cultured, the cells are washed, cultured in a minimum medium containing no nitrogen source, and cultured in a minimum medium containing a nitrogen source for a short time. To this culture fluid, nystatin is directly added and the cells are cultured at 30°c for 1 hour aerobically, then a strain containing ADE1 gene can be preferentially killed. This cell solution is smeared on an appropriate agar medium plate containing adenine and cultured at 30°c for about two days, then a red colony can be obtained.

The obtained adenine-requiring strain can be confirmed by the PCR method, etc. When both terminals of URA3 gene are used as primers, in agarose gel electrophoresis, in the original strain, ADE1 gene, a DNA band having a size of inserted with an ADE1 gene fragment, and DNA having a size of URA3 gene with deletion are detected, but in an ADE1-disrupted strain, DNA having a size of URA3 gene with deletion and a DNA band having a size of URA3 gene with deletion added with a size of ADE1 gene fragment are also detected. At this stage, an ADE1 and URA3 gene-disrupted strain is produced.

### (2) Process for producing an HIS5 gene-disrupted strain, and an HIS5 and ADE1 gene-disrupted strain

An HIS5 gene-disrupted yeast, and an HIS5 and ADE1 gene-disrupted yeast can also be produced from AC16 strain using the method described in the above (1).

HIS5 gene to be used can be prepared from pUC119-HIS5 (Hikiji. et al., Curr. Genet., 16: 261-266 (1989)). That is, DNA for HIS5 gene disruption in which the 5' side DNA fragment of HIS5 gene and 3' side DNA fragment of HIS5 gene are ligated to both terminals of the gene in which the ADE1 gene 5' side fragment is connected to the 3' side of ADE1 gene, etc. can be used (Fig. 1). In the invention, DNA fragments of about 500 bp in the 5' side and 3' side of HIS5 gene was used, but there is not any particular restriction.

A strain in which adenine requirement is recovered by the intramolecular homologous recombination can be easily obtained by electrically introducing the above gene into the AC16 strain, selecting a strain in which HIS5 gene is disrupted from the obtained strain not requiring adenine by the PCR method, etc., and then by carrying out the nystatin concentration method. When a plurality of HIS5 genes occurs, by repeating this process, adenine and histidine double nutrition-requiring strain can be obtained.

### (3) Process for producing a URA3 and HIS5 gene-disrupted strain, and a URA3, HIS5 and ADE1 gene-disrupted strain

Based on the URA3- and ADE1-disrupted strain obtained in (1), by the method described in (2), a URA3 and HISS gene-disrupted strain, and a URA3, HIS5 and ADE1 gene-disrupted strain can be produced. Moreover, the production is also possible using the method of (1) based on the strain obtained in (2).

### (4) Expression of heterogenes by a gene-disrupted strain

Using the gene-disrupted strain obtained in the invention, it becomes possible to introduce an isogene or heterogene for more than once according to the number of utilizable markers, or for any times by recovering the marker, and the target gene can be introduced larger than before, and expressed in a larger amount.

Yeast can secrete glycosylated protein in a medium, differently from Escherichia coli, thus can produce protein using a gene-disrupted strain in such manner. Moreover, in the yeast of the invention, since a plurality of gene markers are occurring, several species of proteins can be expressed, and also a complicated reaction involved with a plurality of enzymes is possible, thus is useful for producing chemical products.

The isogene which can be introduced is not particularly restricted, but as a production example of an industrially useful product, there may be mentioned the production of a dicarboxylic acid by introducing P450 enzyme gene derived from Candida maltosa into the same strain as disclosed in WO 99/04014, for example.

Moreover, the heterogene is not also particularly restricted, but there may be mentioned the production of the protein by introducing an antibody gene, lipase gene, amylase gene, etc., for example. There may also be mentioned the production of polyesters by introducing a polyhydroxyalkanoic acid synthase gene or an enzyme gene synthesizing a substrate for polyhydroxyalkanoic acid synthesis.

The introduction number of the target gene per cell of yeast is determined by the characteristics of the target gene product and the strength of the promoter to be used. For example, in the case that the target gene product from the expression cassette introduced is a simple protein, the introduction number may be any number, but when the protein is modified with a sugar chain, an excessive translation of protein leads to rate-limiting of sugar chain modification, thus gives nonuniform products. Therefore, the expression cassette is preferably introduced in a restricted number.

It is known that in a part of yeasts belonging to the genus Candida such as Candida maltosa used in the invention, the way of translation of codon is partially different from other living organisms in the stage that protein is translated from mRNA. In Candida maltosa, since CUG of leucine codon is translated into serine ( Ohama T. et al, Nucleic Acid Res., 21: 40394045 (1993)), lacZ gene derived from Escherichia coli is not translated into β galactosidase having activity (Sugiyama H. et al, Yeast 11: 43-52 (1995)). In such manner, in the case of expressing a heterogene, there is no guarantee that said heterogene is translated into protein functioning within Candida maltosa. Accordingly, when a heterogene is expressed using Candida maltosa as a host, in principle, leucine codon alone may be converted, but for further efficient expression, other amino acid codon may be adjusted to that of Candida maltosa. The conversion of codon can be carried out with referring to, for example, Candida maltosa p 524-527 written by Mauersberger S. et al. in Nonconventional Yeasts in Biotechnology., edited by Wolf K.

In a preferable embodiment of the present invention, a biodegradable polyester is produced as a gene expression product. In the following, the process for producing a polyester is described.

In the practice of the invention, for example, a plurality of enzyme genes involved with polyester synthesis such as a polyhydroxyalkanoic acid synthase gene (phaC), or an enzyme gene involved with synthesis of a molecule which is to be a substrate for polyester synthesis are incorporated into said gene-disrupted yeast to produce a transformant, and a polyester is harvested from a cultured product obtainable by culturing said transformant.

The enzyme gene involved with the polyester synthesis is not particularly restricted, but is preferably an enzyme gene involved with synthesis of the polyester producible by copolymerizing 3-hydroxyalkanoic acids represented by the following general formula (1), and more preferably an enzyme gene involved with synthesis of copolyester P(3HB-co-3HH) producible by copolymerizing 3-hydroxybutyric acid represented by the following general formula (2) and 3-hydroxyhexanoic acid represented by the following general formula (3).

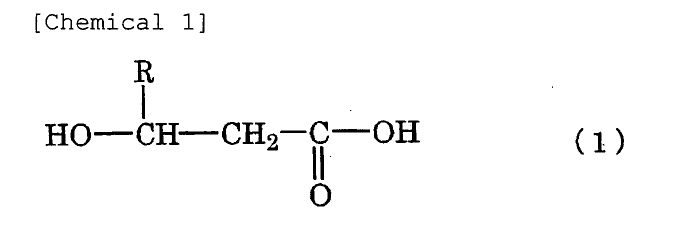

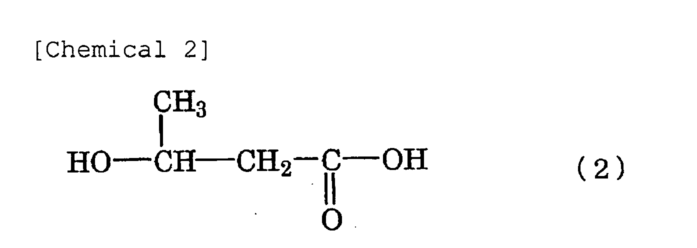

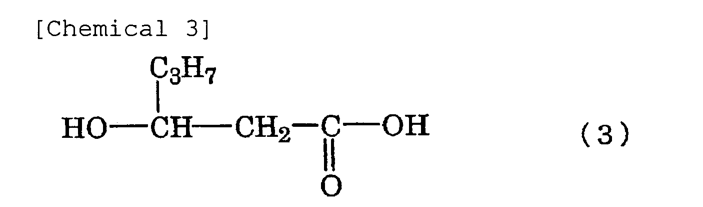

For example, the polyester synthase gene disclosed in Japanese Kokai Publication Hei-10-108682 can be used.

Moreover, together with said polyester synthase gene, a gene involved with (R)-3-hydroxybutyryl-CoA or (R)-3-hydroxybutyryl hexanoyl-CoA, which is to be a substrate for polyester synthesis, can be introduced.

As these genes, there may be mentioned (R)-specific enoyl-CoA hydratase converting enoyl-CoA, which is an intermediate of β oxidization route, into (R)-3-hydroxyacyl-CoA (Fukui T. et al., FEMS Microbiology Letters, 170: 69-75 (1999), and Japanese Kokai Publication Hei-10-108682), β ketothiolase synthesizing 3-hydroxybutyryl-CoA by dimerizing acetyl-CoA, NADPH-dependent reductase gene (Peoples OP et al., J. Biol. Chem. 264: 15298 to 15303 (1989)), and the like. Furthermore, it is also useful to use 3-ketoacyl-CoA-acyl career protein reductase gene (Taguchi K. et al., FEMS Microbiology Letters, 176: 183-190 (1999)).

As long as they have substantial enzyme activities, there may be mutations such as deletion, substitution and insertion in the base sequences of said genes. However, in the case of heterogenes, since there is no guarantee of being translated into proteins functioning within Candida maltosa as mentioned above, it is desirable to change the amino acid codon.

More preferably, a polyester synthase gene and acetoacetyl-CoA reductase gene can be used in combination.

In the present invention, phaC derived from Aeromonas caviae (Japanese Kokai Publication Hei-10-108682, and Fukui T. et al., FEMS Microbiology Letters, 170: 69-75 (1999)), and phbB derived form Ralstonia eutropha (GenBank: J04987) were used.

The base sequence of DNA (phaCac149NS) produced in such manner of a gene coding for phaC derived form Aeromonas caviae being designed so as to express within Candida maltosa, and asparagine occurring in 149th from the amino terminal on the amino acid sequence being substituted with serine was shown under SEQ ID No:2.

The base sequence of DNA in which a gene coding for phbB derived form Ralstonia eutropha was designed so as to express within Candida maltosa was shown under SEQ ID No:3.

However, the base sequences shown under these SEQ ID Nos. are not limited to these, and any of base sequences of which amino acid sequence of said enzymes are expressed within Candida maltosa can be used. More preferably, one added with a gene coding for sequences comprising three amino acid residues, namely "(serine/alanine/cysteine)-(lysine/arginine/histidine)-leucine" to carboxyl terminals of these genes involved with polyester synthesis as a peroxisome-targeting signal can be used. Herein, for example, "(serine/alanine/cysteine)" represents either of serine, alanine, or cysteine (WO 03/033707).

The gene expression cassette in yeast is produced by ligating a DNA sequence such as a promoter and 5' upstream region activation sequence (UAS) on the 5' side upstream of said gene, and ligating a DNA sequence such as a poly A addition signal and terminator on the 3' downstream of said gene. Any sequences can be used provided that it can function within said yeast as these DNA sequences.

Any promoter and terminator sequences may be used provided that they can function in yeast. While, among the promoters, there are ones causing constitutive expression and ones causing inducible expression, either type of promoter may be used. In the practice of the present invention, it is preferable that the promoter and terminator can function in Candida maltosa, and it is more preferable that the promoter and terminator are derived from Candida maltosa. Still more preferred is a promoter having strong activity in the carbon source to be used.

When, for example, fats and oils, etc. are used as the carbon source, as a promoter, promoter ALK1p (WO 01/88144) of ALK1 gene of Candida maltosa (GenBank: D00481), promoter ALK2p of ALK2 gene (GenBank: X55881), and the like can be used. Furthermore, the promoter improved with promoter activity by adding multiple ARR (alkane responsible region) sequences to the upstream of these promoters (Kogure et al., Summaries of Japan Agricultural Chemical Convention Lecture in 2002, p 191) (SEQ ID No:4) can also be used. As the terminator, terminator ALK1t of ALK1 gene of Candida maltosa (WO 01/88144), and the like can be used.

In addition, the base sequence of the above promoter and/or terminator may have one or a plurality of bases being deleted, substituted and/or added provided that it can function within Candida maltosa.

The promoter is ligated to the 5' upstream of the gene coding for the enzyme involved with polyester synthesis with an added DNA coding for a peroxisome-targeting signal, and the terminator is ligated to the 3' downstream of the gene coding for enzyme involved with polyester synthesis with the added DNA coding for a peroxisome-targeting signal, respectively.

The method of constructing the gene expression cassette according to the invention by joining the promoter and terminator to the structural gene is not particularly restricted. Except for ones represented in the example section to be described below, of the invention, the PCR method can be utilized in order to form appropriate restriction sites. The method described in WO 01/88144 can be used, for example.

In order to incorporate this expression cassette into a yeast chromosome site-specifically, DNA (DNA for introduction) in which gene fragments having homologous sequences with the chromosomal gene to be introduced are jointed at the both terminals of DNA in which the expression cassette and a gene to be a selective marker can be used. As the selective marker, it is also possible to use ADE1 gene, etc. capable of being spontaneously deleted by the intramolecular homologous recombination as described in the above (1). There is no restriction in the number of the expression cassette in DNA for introduction, and any numbers are allowable provided that production can be conducted.

As a site for inserting the target gene, any sites can be used provided that the gene sequence is elucidated. Even if the gene sequence is unknown, since it is possible to analyze the gene sequence on the basis of the chromosomal gene sequence of related yeast species of which the gene sequence is known, substantially the insertion into all of gene sites is possible. The gene sequence can be analyzed from a DNA library of the introduction object yeast chromosome, using the homologous gene fragment of the genus Saccharomyces cerevisiae or Candida albicans, the sequence of which has been already analyzed, as a probe, and by carrying out a hybridization. The probe can be produced using PCR, etc. The chromosomal DNA library can be produced by the method well-known to a person skilled in the art.

As an example, HIS5 gene is inserted as a marker gene into DNA-1 for URA3 disruption used for disrupting URA3 gene as described in (1), and the expression cassette of the gene involved with polyester synthesis between the above inserted site and the URA3 gene fragment site, DNA for inserting the target gene specifically to URA3 site disrupted on the yeast chromosome can be produced using histidine requirement as a marker. As the introduction method of the gene, electric introduction method described in (1), etc. can be used.

The strain of the present invention can produce various strains introduced with a plurality of gene expression cassettes by utilizing a plural selective markers and carrying out transformation. When ADE1 gene, etc., which is spontaneously deletable by the intramolecular homologous recombination, is used, gene introduction into many sites is possible since the selective markers can be regenerated. Moreover, plasmids capable of automonous replication in yeast can also be combinedly used.

Production of polyesters by culturing yeast transformed with a gene expression cassette involved with polyester synthesis can be carried out as follows.

Any carbon sources can be used for the culture provided that yeast can assimilate. When the promoter expression is of the inducible type, an inducer is to be added appropriately. In some instances, the inducer may serve as the main carbon source. As the nutrition source except for the carbon sources, media containing a nitrogen source, an inorganic salt, other organic nutrient sources and the like can be used, for example. The culture temperature may be within a temperature range in which the organism can grow, preferably 20°C to 40°C. The culture time is not particularly restricted and may be about 1 to 7 days. Then, polyesters can be harvested from the obtained cultured cells or cultured product.

As a preferable embodiment of the present invention, as the carbon source, fats and oils, fatty acids, alcohols, and further n-alkane, etc. can be used. As the fats and oils, there may be mentioned rapeseed oil, coconut oil, palm oil and palm kernel oil, etc. As the fatty acids, there may be mentioned butanoic acid, hexanoic acid, octanoic acid, decanoic acid, lauric acid, oleic acid, palmitic acid, linolic acid, linolenic acid, myristic acid, and like saturated and unsaturated acids, as well as esters and salts of these fatty acids and other fatty acid derivatives, and the like. These may also be mixed and used in combination. In the case of fats and oils, which cannot be assimilated efficiently or at all, improvements can be achieved by adding lipase to the medium. Furthermore, yeast can be provided with the ability to assimilate fats and oils by transformation with a lipase gene.

As the nitrogen source, there may be mentioned, for example, ammonia, ammonium chloride, ammonium sulfate, ammonium phosphate, and other ammonium salts, as well as peptone, meat extract, yeast extract, and the like.

As the inorganic salts, there may be mentioned, for example, potassium dihydrogenphosphate, dipotassium hydrogenphosphate, magnesium phosphate, magnesium sulfate, sodium chloride, and the like.

As the other organic nutrient sources, there may be mentioned, for example, amino acids such as glycine, alanine, serine, threonine, proline and the like; vitamins such as vitamin B1, vitamin B12, biotin, nicotinamide, pantothenic acid, vitamin C and the like; and the like.

As the inducer, there may be mentioned glucose, galactose, etc.

As the harvest of polyesters from cells, many methods have been reported. In the practice of the invention, for example, the following methods can be used. After completion of culture, cells are separated and collected from the culture fluid using a centrifuge and the like and the cells are washed with distilled water and methanol or the like, and then dried. At this stage, a process for disrupting the cells can be added. The polyester is extracted from these dried cells using an organic solvent such as chloroform and the like. The cell fraction is removed from the organic solvent solution containing the polyester by filtration and the like. A poor solvent, such as methanol, hexane or the like, is added to the filtrate to cause the polyester to precipitate out. Then, the supernatant is removed by filtration or centrifugation, and precipitate polyester is dried. The polyester can be thus harvested.

The polyester obtained can be analyzed by, for example, gas chromatography, nuclear magnetic resonance spectrometry and/or the like.

Next, the novel yeast transformant which is the second aspect of the present invention is described.

The second aspect of the present invention relates to a yeast transformant
which is introduced with a polyhydroxyalkanoic acid synthase gene and an acetoacetyl CoA reductase gene, and both or either of these genes being introduced in 2 or more copies.

### (I) Host

The "yeast" so referred to in the second aspect of the invention may be any of those capable of introducing and transforming a plurality of genes, and yeasts deposited with organism depositories (e.g. IFO, ATCC, etc.) can be used. Preferably, in view of having resistance to hydrophobic substances such as a straight-chain hydrocarbon, usable are yeast of the genus Candida, the genus Clavispora, the genus Cryptococcus, the genus Debaryomyces, the genus Lodderomyces, the genus Pichia, the genus Rhodotorula, the genus Sporidiobolus, the genus Stephanoascus, the genus Yarrowia, and the like.

Among these yeasts, those belonging to the genus Candida are more preferred from the viewpoints that the analysis of chromosomal gene sequence is advanced, host-vector system is also applicable, and assimilation ability of a straight-chain hydrocarbon, fats and oils, etc. is high.

Among yeasts belonging to the genus Candida, particularly in view of having high assimilation ability of a straight-chain hydrocarbon, fats and oils, etc., ones exemplified in the first aspect of the present invention are preferably used.

Among these species, particularly ones of the maltosa species are preferred in view of the growth rate and infectivity.

In producing the yeast transformant of the present invention, when a selective marker gene having drug resistances, nutritional requirement, and the like characteristics is introduced into a host concurrently with carrying out a transformation, it becomes possible to select the transformant alone using drug resistances, nutritional requirement, etc. exhibited by the expression of the selective marker gene after the transformation.

As the selective marker gene, genes providing resistance to cycloheximide, G418 or Hygromycin B, etc. can be used. Moreover, a gene complementing nutritional requirement can also be used as a selective marker. These may be used alone, or two or more of them may also be used in combination.

However, when using the gene complementing nutritional requirement is used as the selective marker, for example, a nutritional requirement-disrupted strain is required which has the same function as the selective marker and in which genes originally occuring in the host does not substantially function. Such nutritional requirement-disrupted strain (mutant strain) can be obtained by a random mutagenesis treatment using a mutagen such as nitrosoguanidine and ethylmethane sulfonate. However, there is high possibility that the part other than the target one may be mutated and as a result the growth rate, etc. may be affected in some cases. Thus, in the practice of the invention, it is rather preferable to use the host produced by gene disruption by the homologous recombination as mentioned in the first aspect of the present invention.

In addition, when the transformation is carried out for more than once, marker species are required according to the number of times. Or, as mentioned below, it is necessary to recover the selective marker by removing the selective marker gene after introducing DNA for gene introduction containing the selective marker gene into a host. In the examples of the present invention, a multiple nutrition requiring gene-disrupted strain was used.

Herein, "DNA for gene introduction" refers to DNA which can cause the homologous recombination with a gene on a chromosome within microbial cells, and thereby can insert the target gene.

The Candida maltosa AHU-71 strain used in the examples of the present invention, which is an ADE1, HIS5, and URA3 gene-disrupted strain, is one produced in the first aspect of the invention, and produced by the method described in Example 3 mentioned below using the Candida maltosa AC16 strain.

Particularly in the above host, when using one with which a plurality of selective marker genes can be used from the first without disrupting genes having nutritional requirement, etc. in carrying out the transformation for more than once, the yeast transformant of the present invention can be efficiently produced.

### (II) PHA synthase gene and acetoacetyl CoA reductase gene

The PHA synthase gene is not particularly restricted, but preferred is a synthase gene synthesizing polyesters produced by copolymerizing 3-hydroxyalkanoic acid represented by the above general formula (1), and more preferred is a synthase gene of copolyester P(3HB-co-3HH) produced by copolymerizing 3-hydroxybutyric acid represented by the above formula (2) and 3-hydroxyhexanoic acid represented by the above formula (3).

As the PHA synthase gene, for example, the PHA synthase gene disclosed in Japanese Kokai Publication Hei-10-108682 can be used.

Moreover, in the second aspect of the present invention, an acetoacetyl-CoA reductase gene is used combinedly with the above PHA synthase gene. The acetoacetyl-CoA reductase gene may be an enzyme gene reducing acetoacetyl-CoA and having activity synthesizing (R)-3-hydroxybutyryl-CoA, and for example, enzyme genes derived from Ralstonia eutropha (GenBank: AAA21973), Pseudomonas sp.61-3 (GenBank: T44361), Zoogloea ramigera (GenBank: P23238), Alcaligenes latus SH-69 (GenBank: AAB65780), and the like can be used.

Furthermore, in addition to the above-mentioned genes, other genes involved with PHA synthesis can be used. As the other genes involved with PHA synthesis, for example, there may be mentioned
(R)-specific enoyl-CoA hydratase converting enoyl-CoA, which is an intermediate of β oxidization route, into (R)-3-hydroxyacyl-CoA (Fukui T. et al., FEMS Microbiology Letters, 170: 69-75 (1999), and Japanese Kokai Publication Hei-10-108682), β ketothiolase synthesizing 3-hydroxybutyryl-CoA by dimerizing acetyl-CoA (Peoples OP et al., J. Biol. Chem. 264: 15298-15303 (1989)), 3-ketoacyl-CoA-acyl career protein reductase gene (Taguchi K. et al., FEMS Microbiology Letters, 176: 183-190 (1999)), and the like. Particularly preferred is an enzyme gene having a synthesis activity of (R)-3-hydroxyhexanoyl CoA.

In the present invention, a PHA synthase gene (phaC) and acetoacetyl-CoA reductase gene (phbB) are concurrently used.

In the practice of the invention, phaC derived from Aeromonas caviae (Japanese Kokai Publication Hei-10-108682, and Fukui T. et al., FEMS Microbiology Letters, 170: 69-75 (1999)) and phbB derived form Ralstonia eutropha (GenBank: J04987) can be used. As phaC mentioned above, preferred is one coding for an enzyme or mutant derived from Aeromonas caviae having the amino acid sequence shown under SEQ ID No:5, and as phbB mentioned above, preferred is one coding for an enzyme or mutant derived from Ralstonia eutropha having the amino acid sequence shown under SEQ ID No:6.

As long as they have substantial enzyme activity, there may be mutations such as deletion, substitution and insertion in the base sequences of said genes. However, in the case of heterogenes, since there is no guarantee of being efficiently translated into proteins functioning within the host yeast, it is desirable to optimize the amino acid codon.

As mentioned in the first aspect of the present invention above, when heterogenes are expressed using Candida maltosa as a host, in principle, it is preferable to convert leucine codon (CUG) alone, and for further efficient expression, other amino acid codon may be adjusted to that of Candida maltosa.

It is possible to convert the amino acid sequence of a PHA synthase gene to obtain/produce and utilize a mutant improved with characteristics such as enzyme activity, substrate specificity and thermal stability. Various useful mutation methods are known, but especially, means utilizing molecular evolution technology (Japanese Kokai Publication 2002-199890) and the like are highly useful because desired mutants can be obtained in a short time. Utilizing these technologies, several synthase mutants have been found in the past, which were confirmed to have more improved activity than that of wild type enzymes in Escherichia coli (T. Kichise et al., Appl. Environ. Microbiol. 68, 2411-2419 (2002), Amara A. A. et al., Appl. Microbiol. Biotechnol. 59, 477-482 (2002)).

It is also possible to identify useful amino acid mutations on the basis of an enzyme structure or an estimated structure thereof by computing, for example, by using program Shrike (Japanese Kokai Publication 2001-184831) and the like. As the phaC in the present invention, for example, one coding for a PHA synthase mutant obtainable by utilizing these technologies and by applying at least one of the following amino acid substitutions from (a) to (h) to an Aeromonas caviae-derived PHA synthase gene;
(a) substitution of Ser for Asn-149
(b) substitution of Gly for Asp-171
(c) substitution of Ser or Gln for Phe-246
(d) substitution of Ala for Tyr-318
(e) substitution of Ser, Ala or Val for Ile-320
(f) substitution of Val for Leu-350
(g) substitution of Thr, Ser or His for Phe-353
(h) substitution of Ile for Phe-518.

In this description, for example, "Asn-149" means asparagine located at 149th position in the amino sequence shown under SEQ ID No:5, and amino acid substitution (a) means a conversion of asparagine located at 149th position into serine.

As the phaC and phbB, there may be mentioned but are not limited to those having the sequences shown under SEQ ID No:2 and 3 exemplified in the first aspect of the present invention, and any base sequences can be used provided that the amino acid sequence of said enzyme gene is expressed within Candida maltosa.

The phaC and phbB are used as such when they are caused to express in cytosol, but it is also possible to use these genes by converting to genes localized in peroxisome (WO 03/033707).

As a method for causing localization of the phaC and phbB in peroxisome, the method described in the first aspect of the invention can be used.

Further, sequences occurring in the vicinity of the N terminus and comprising 9 amino acid residues, namely "(arginine/lysine)-(leucine/valine/isoleucine)-(5 amino acid residues)-(histidine/glutamine)-(leucine/alanine)", are also known as peroxisome-targeting signals. By inserting and adding DNA coding for these sequences into the gene involved with PHA synthase, it is possible to cause localization of the enzyme gene in peroxisomes.

Furthermore, for causing the phaC and phbB to express within mitochondria, these genes can be used by converting into those targeting to mitochondria. For causing localization of these genes in mitochondria, protein localized and expressed in mitochondria may be coupled to an amino terminal. For example, there may be mentioned cytochrome oxidase, TCA cycle-related enzyme, and the like. For example, the gene coupled with a gene coding for 15 or more residues, desirably 40 or more residues from the amino terminal of the protein localized and expressed in mitochondria to the 5' side upstream of the gene involved with PHA synthesis so as not to be frameshifted construct. It is also possible to insert a linker sequence between a fusion gene to be added at this stage and the gene involved with PHA synthesis for preventing unnecessary collision of amino acid residues. The fusion gene to be used is preferably derived from the host yeast used for the transformation in the invention, but there is no particular limitation.

These genes designed to target to cytosol, peroxisome, and mitochondria may be used alone, or two or more of them may also be used.

As the phaC and phbB, those added with a peroxisome-targeting signal are preferred.

### (III) Gene expression cassette

The expression cassette of a PHA synthase gene and acetoacetyl CoA reductase gene used for the present invention can be produced by ligating such DNA sequences as a promoter, the upstream activating sequence (UAS), etc. to the 5' side upstream of the gene and ligating such DNA sequences as poly(A) additional signal, terminator, etc. on the 3' downstream of the gene.

In the invention, it is preferable that a promoter and terminator functioning in yeast are connected to the PHA synthase gene and acetoacetyl CoA reductase gene.

Any promoter and terminator sequences may be used provided that they can function in yeast. While, among the promoters, there are ones causing constitutive expression and ones causing inducible expression, either type of promoter may be used. As the promoter, one having a strong activity on the carbon source used for culturing the transformant. For example,' when fats and oils, etc. are used as the carbon source, such as those described in the first aspect of the invention can be used.

Moreover, the terminator ALK1t (WO 01/88144) of the Candida maltosa ALK1 gene and the like terminator can be used as the terminator. The base sequences of the above promoters and/or terminators each may be the base sequences in which one or a plurality of nucleotides may have undergone deletion, substitution and/or addition provided that they can function in the host to be used.

In the practice of the present invention, it is desirable that the promoter and terminator can function in the genus Candida, more desirably in Candida maltosa, and still more desirably the promoter and terminator are derived from Candida maltosa.

In a preferable embodiment of the invention, the promoter is ligated to the PHA synthase gene added with DNA coding for a peroxisome-targeting signal, and to the 5' upstream of acetoacetyl CoA reductase gene added with DNA coding for a peroxisome-targeting signal. The terminator is ligated to the PHA synthase gene added with DNA coding for a peroxisome-targeting signal, and to the 3' downstream of acetoacetyl CoA reductase gene added with DNA coding for a peroxisome-targeting signal (WO 03/033707).

The method of constructing the gene expression cassette according to the present invention by joining the promoter and terminator to the phaC and phbB is not particularly restricted, and the same method according to the first aspect of the invention can be used.

### (IV) Transformant

According to the invention, it was shown that although gene expression is strongly induced under the carbon source such as hydrocarbons, fatty acids, fats and oils, etc., the introduction number of the above expression cassette per cell of yeast is insufficient for 1 copy even when the ARR promoter, which was used in the preferable embodiment of the invention, was used, and 2 or more copies of either the phaC or phbB expression cassette numbers are necessary. There is no restriction for the number of expression cassette to be introduced as long as the supplied amount of substrate in PHA synthesis of the host does not restrict the enzyme reaction, and the larger number is preferable. The preferable number of the expression cassette depends on the species of the promoter to be used, but when promoter ARRp is used, both are preferably introduced in 2 or more copies, and more preferably 3 or more copies.

This expression cassette can be introduced into a host yeast by inserting to a vector capable of autonomous replication within yeast. Moreover, it can also be inserted into a host yeast chromosome. Both introduction methods can also be used at the same time.

For the introduction into a host yeast using a vector, for example, an expression vector in which a plurality of expression cassettes are introduced such as pUTU1 capable of autonomous replication in Candida maltosa (M. Ohkuma, et al., J. Biol. Chem., vol. 273, 3948-3953 (1998)) may be produced.

When a method of inserting the expression cassette into a chromosome is used, for example, the homologous recombination can be used. Among the homologous recombination, a gene substitution method is preferable since an introduced strain which is not spontaneously reverted can be obtained. For inserting an expression cassette into a chromosome by a gene substitution method, DNA (DNA for gene introduction) can be used which can be prepared by coupling the expression cassette and a gene which is to be a selective marker firstly, and then coupling a gene fragment having the homologous sequence with the gene on the chromosome which is to be introduced to both terminals of the resultant DNA.

The site for inserting an expression cassette, etc. on the chromosome is not particularly restricted provided that the host is not affected irreversible damage. The length of the homologous region between the gene on the chromosome to be introduced coupled to both terminals of DNA for gene introduction is preferably 10 bases or more, more preferably 200 bases or more, and still more preferably 300 bases or more. The homology of the respective terminals is preferably 90% or more, more preferably 95% or more. That is, in the site of which the gene sequence has been analyzed, said gene can be used as such, and even when the gene sequence is unknown, the chromosomal gene sequence of related yeast species of which the gene sequence is known can be used.

In the case where it is difficult to cause the homologous recombination due to low homology, it is possible to use a gene on the introduction site on the chromosome by cloning. For cloning the gene on the introduction site on the chromosome, a primer for PCR may be designed on the basis of the sequences of Saccharomyces cerevisiae or Candida albicans, all of whose sequences of chromosomal genes have been analyzed, and gene amplification may be carried out. Furthermore, in the same manner as in the first aspect of the invention, it is also possible to use a chromosomal DNA library of the introduction object yeast.

The number of expression cassettes in DNA for gene introduction is not limited, and any number is allowable provided that the production is possible.

As the selective marker gene in DNA for gene introduction, the gene complementing nutritional requirement can be used as a selective marker gene as mentioned above. Moreover, a resistance-providing gene such as cycloheximide, G418 or Hygromycin B can also be used. These selective marker genes can also be used in the form capable of spontaneous deletion by the below-mentioned intramolecular homologous recombination. In this case, since it is possible to recover the selective marker gene, DNA for gene introduction using the same selective marker gene can be introduced for any of times, and the transformed strain can be produced with ease.

The DNA for gene introduction for introducing these expression cassettes into a yeast host can be produced by a method well-known to a person skilled in the art using a plasmid capable of autonomous proliferation in Escherichia coli, etc. As an example, HIS5 gene is inserted into DNA-1 for URA3 disruption described in Example 1 below as a selective marker gene, and then the expression cassette of phaC and the expression cassette of phbB are inserted between the above inserted site and the URA3 gene fragment site, DNA for gene introduction for inserting the target gene specifically to URA3 site on a yeast chromosome can be produced by using histidine requirement as a marker.

The plasmid containing DNA for gene introduction can be prepared by the same method as in the first aspect of the invention. Although this plasmid can be used directly for transforming yeast, it is desirable to cut a portion having homology and containing a chromosome-introduced region from the purified vector with an appropriate restriction enzyme, and use the resultant as DNA for gene introduction. It is also possible to amplify the portion using the PCR method, and use the same.

The method exemplified in the first aspect of the invention can be mentioned for the transformation method of yeast, and the electric pulse method is preferable in the present invention. The method comprises preparing competent cells from a host strain, subjecting the cells to electric pulse together with DNA for gene introduction, culturing the resultant in a medium in which a teransformant not containing a selective marker gene is not proliferated, and then screening a strain inserted with DNA for gene introduction to the target chromosome site from the appeared colony.

Screening of the target gene-introduced strain can also be carried out in the same manner as in the first aspect of the invention.

The transformant of the invention can be produced by introducing the phaC expression cassette and phbB expression cassette by the above method until the number of cassettes becomes to the object expression cassette number.

It is also possible to use a wild strain or a strain having only one nutritional requirement instead of using a multiple nutrition-requiring gene-disrupted strain used in the examples of the invention to produce the yeast transformant introduced with the gene involved with PHA synthesis of the invention more than once. For example, when DNA for gene introduction is introduced using a drug-resistance marker as the selective marker gene, the concentration of the drug used for selecting the transformant may be increased at every stage that the transformation is carried out with DNA for gene introduction. Moreover, when the selective marker gene introduced into a chromosome is removed after one transformation, it can be used as a marker for gene introduction again, thus many of DNA for gene introduction can be introduced. As this method, for example, the gene disruption method disclosed in Japanese Kokai Publication 2002-209574, and the like can be used. Furthermore, by inserting a hisG gene fragment to both terminals of the selective marker gene, DNA for gene introduction can be produced in the form that the marker gene inserted by the intramolecular homologous recombination is removable after introducing a gene (Alani et al., Genetics, 116: 541-545(1987)).

The CM313-X2B strain (accession number: FERM BP-08622), which is one of the polyester-producing strains obtained in the invention has been internationally deposited with the National Institute of Advanced Industrial Science and Technology on the Budapest Treaty on February 13, 2004.

### (V) Method for recovering a selective marker

The method for recovering the selective marker of the present invention comprises removing ADE1 gene by carrying out the intramolecular homologous recombination in Candida maltosa which has ADE1 gene as a selective marker gene. By removing said ADE1 gene, ADE1 gene can be used as the selective marker gene again when further transformation is carried out.

Hitherto, it has been well known that a selective marker gene is removed by carrying out the intramolecular homologous recombination in Saccharomyces cerevisiae, etc. but a method of removing a selective marker gene by means of intramolecular homologous recombination in Candida maltosa has not been known. As the selective marker gene, a gene complementing drug resistances and nutritional requirements can be used as mentioned above, and for example, there may be mentioned ADE1 gene, URA3 gene, HIS5 gene, etc. In the present invention, ADE1 gene is used which can be selected by colors in removing a selective marker gene.

In the method of the present invention, said ADE1 gene can be removed by the intramolecular homologous recombination even from one having a homologous gene being coupled. However, one having a part of ADE1 gene being coupled to the upstream or downstream of ADE1 gene is preferred since the production is easy and excess genes are not remained on a yeast chromosome.

The gene fragment used for the intramolecular homologous recombination of a selective marker gene is not particularly restricted and a gene fragment with which the selective marker gene does not substantially function may be used. In the examples of the invention, a gene fragment of the 5'-terminal portion of ADE1 gene was used, but a gene fragment of the 3'-terminal portion can also be used. The marker gene fragment to be ligated to the selective marker gene preferably has 10 bases or more, more preferably 200 bases or more, and still more preferably 300 bases or more. That is, to the 5' terminal or 3' terminal of the selective marker gene in DNA for gene introduction described in the above (IV), the marker gene fragment may be inserted. In the method of the invention, ADE1 gene preferably has the base sequence shown under SEQ ID No:7. The base sequence shown under SEQ ID No:7 is derived from Candida maltosa. This method can also be applied to a marker gene other than ADE1 gene.

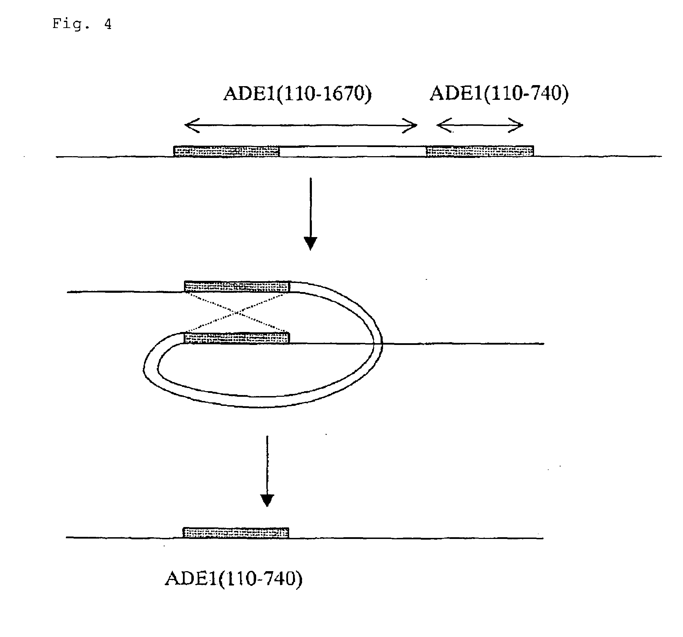

In Fig. 4, a diagram of marker recovery by the intramolecular homologous recombination is shown. In Fig. 4, the numbers in parentheses represent the number from the 5' terminal of the sequence registered on GenBank of ADE1 gene.

By the intramolecular homologous recombination, the strain removed with an inserted selective marker gene can be concentrated and selected by various methods. For example, a nystatin concentration method can be used. Cells cultured in an appropriate medium are sown and cultured in a minimum medium, etc. The cells are washed and cultured in a minimum medium containing no nitrogen source, and cultured in a minimum medium containing a nitrogen source for a short time. To this culture fluid, nystatin is directly added and cultured for 1 hour at 30°c aerobically, thereby a strain having a marker gene can be preferentially killed. The cell solution is smeared on an appropriate agar medium plate, and cultured for about 2 days at 30°c. When the selective marker gene to be removed is ADE1 gene showing adenine-requirement, since when ADE1 gene is disrupted, a precursor substance is accumulated and yeast is dyed red, the yeast can be obtained as a red colony when an adenine-containing minimum medium agar plate is used. When the marker gene is URA3 gene, a colony growing in a medium under the coexistence of uridine or uracil and 5-FOA (5-fluoro-orotic-acid) may be selected. When there is no such selection method, a replica method can be used.

### (VI) Controlling method of physical properties of polyesters

Moreover, the method for controlling the molecular weight of a polyester according to the invention comprises controlling the number of an acetoacetyl CoA reductase gene in the yeast transformant in the production of polyesters using the yeast transformant.

Furthermore, the method for controlling the composition of a hydroxyalkanoic acid of a polyester according to the invention comprises controlling the number of polyhydroxyalkanoic acid synthase gene in the yeast transformant in the production of polyesters using the yeast transformant.

That is, the hydroxyalkanoic acid composition and the molecular weight of a polyester, which is the object product of the invention, can be controlled by adjusting the expression amounts of the phaC and phbB. When the expression cassette of phaC and the expression cassette of phbB using respectively the same promoter are used, by raising the number of introduction of the expression cassette of phaC relative to that of phbB, the composition of a hydroxyhexanoic acid can be increased. In addition, by raising the number of introduction of the expression cassette of phbB relative to that of phaC, the molecular weight can be increased.

The transformant having such characteristics can be produced by the method described in the above (IV). Moreover, even when the numbers of introduction of the expression cassette are the same, the composition and molecular weight of a hydroxyalkanoic acid can be controlled by changing the strength of the promoter to be used.

### (VII) Culture purification

The process for producing a polyester according to the present invention comprises harvesting a polyester from a cultured product obtained by culturing the above yeast transformant.

The culture of a yeast transformed with a PHA synthase gene and an expression cassette of phbB can be carried out by the culture method of the transformed yeast in the same manner as described in the first aspect of the present invention.

Many methods have been reported for harvesting polyesters from cells, and for example, the method described in the first aspect of the present invention can be used.

The polyester obtained is analyzed by, for example, gas chromatography, nuclear magnetic resonance spectrometry and/or the like. Weight average molecular weight can be determined by GPC method. For example, harvested dried polymers are dissolved in chloroform, and then this solution may be analyzed by Shimadzu Corporation's GPC system equipped with Shodex K805L (product of Showa Denko K. K.) using chloroform as a mobile phase. Commercial standard polystyrene and the like may be used as the standard molecular weight sample.

### BRIEF DESCRIPTION OF THE DRAWINGS

Fig. 1 represents a simple diagram showing DNA for disruption produced in Examples.
Fig. 2 represents a graph showing a comparison of the proliferation ability of a novel gene-disrupted yeast.
Fig. 3 represents a diagram showing DNA-1 to 4 for gene introduction produced and used in Example 4.
Fig. 4 represents a diagram showing the intramolecular homologous recombination.

### BEST MODE OF CARRYING OUT THE INVENTION

The following examples illustrate the present invention more specifically. These examples are, however, by no means limitative of the technical scope of the present invention.

In addition, unless otherwise specified, the reagents used in yeast cultivation were commercial products available from Wako Pure Chemical Industries.

Moreover, in the practice of the present invention, many species of kit were used, but unless otherwise specified, they were used according to the attached instruction.

### (Medium composition)

LB medium: 10 g/L tripton, 5 g/L yeast extract, 5 g/L sodium chloride. In the case of an LB plate, agar is added so as to be 16 g/L.

YPD medium: 10 g/L yeast extract, 20 g/L polypeptone, 20 g/L glucose. In the case of a YPD plate, agar is added so as to be 20 g/L. In the case of an adenine-containing YPD medium, adenine is added in 0.1 g/L.

YM medium: 3 g/L yeast extract, 3 g/L malt extract, 5 g/L bactopeptone, 10 g/L glucose.

SD medium: 6.7 g/L yeast nitrogen base not containing amino acid (YNB), 20 g/L glucose. In the case of an adenine-containing medium, adenine is added in 24 mg/L. In the case of a uridine-containing medium, uridine is added in 0.1 g/L. In the case of a histidine-containing medium, histidine is added in 50 mg/L. In the case of an SD plate, agar is added so as to be 20 g/L.

M medium: 0.5 g/L magnesium sulfate, 0.1 g/L sodium chloride, 0.4 mg/L thiamin, 0.4 mg/L pyridoxine, 0.4 mg/L calcium pantothenate, 2 mg/L inositol, 0.002 mg/L biotin, 0.05 mg/L iron chloride, 0.07 mg/L zinc sulfate, 0.01 mg/L boric acid, 0.01 mg/L copper sulfate, 0.01 mg/L potassium iodide, 87.5 mg/L potassium dihydrogenphosphate, 12.5 mg/L dipotassium hydrogenphosphate, 0.1 g/L calcium chloride, 20 g/L glucose. In the case of an ammonium sulfate-containing M medium, 1 g/L ammonium sulfate is added. In the case of an ammonium sulfate- and adenine-containing M medium, 1 g/L of ammonium sulfate and 24 mg/L of adenine are added to M medium. In the case of an ammonium sulfate-, adenine- and uridine-containing M medium, 1 g/L of ammonium sulfate, 24 mg/L of adenine, and 0.1 g/L of uridine are added to M medium. In the case of an ammonium sulfate-, adenine- and histidine-containing M medium, 1 g/L of ammonium sulfate, 24 mg/L of adenine, and 50 mg/L of histidine are added to M medium. In the case of an ammonium sulfate- and adenine-, uridine- and histidine-containing M medium, 1 g/L of ammonium sulfate, 24 mg/L of adenine, 0.1 g/L of uridine, and 50 mg/L of histidine are added to M medium.

M2 medium (12.75 g/L ammonium sulfate, 1.56 g/L potassium dihydrogenphosphate, 0.33 g/L dipotassium hydrogenphosphate trihydrate, 0.08 g/L potassium chloride, 0.5 g/L sodium chloride, 0.41 g/L magnesium sulfate heptahydrate, 0.4 g/L calcium nitrate heptahydrate, and 0.01 g/L Iron(III) trichloride tetrahydrate) are supplemented with 2 w/v % palm oil and 0.45 ml/L of trace elements dissolved in hydrochloric acid (1 g/mL Iron(II) sulfate heptahydrate, 8 g/mL zinc(II) sulfate heptahydrate, 6.4 g/mL manganese(II) sulfate tetrahydrate, and 0.8 g/mL cuprous(II) sulfate pentahydrate). As the carbon source, 20 g/L of fats and oils is added.

Liquid culture of yeast was carried out using a 50 ml test tube, 500 ml Sakaguchi flask, and 2L Sakaguchi flask or mini jar. In the respective cases using a 50 ml test tube, 500 ml Sakaguchi flask, and 2L Sakaguchi flask, the shaking culture was carried out at a rate of 300 rpm, 100 to 110 rpm, and 90 to 100 rpm, respectively. The culture temperature was 30°c in the both cases of liquid culture and plate culture.

### (Restriction enzyme treatment)

The restriction enzyme treatment was carried out under the reaction conditions recommended by manufacturer or by the method described in Molecular cloning, edited by Sambrook, etc.: A Laboratory Manual, Second Edition, Cold Spring Harbor Laboratory Press (1989).

### (Example 1) Production of a complementary vector, gene for disruption, and marker recovery type gene for disruption

Vector pUTU-delsal in which SalI restriction enzyme site is disrupted in URA3 gene was produced from pUTU-1, which is a vector for Candida maltosa having URA3 gene as a marker imparted from Tokyo University, by using the primers (del-sal-5, del-sal-3) as shown under SEQ ID No:8 and 9 with a quick change kit produced by Stratagene. From this pUTU-delsal, URA3 gene was cut with SalI and XhoI, and plasmid pUTU-2 in which the cut fragment was introduced into XhoI site of pUTU-1 again was produced. ADE1 gene on pUTA-1 (disclosed in WO 01/88144) was cloned to XhoI site of pUTU-2 to produce pUTU-2-Ade. To SalI site of a multicloning site of pUTU-1, HIS5 gene cloned to pUC119 was cloned to produce pUTU1-His. Furthermore, to SalI site of a multicloning site of pUTU-2-Ade, HIS5 gene was cloned to produce pUTU-2-Ade-His.

To SphI-SalI site of plasmid pUC-Nx prepared by converting a multicloning site of pUC19 to NotI-SphI-SalI-XhoI-NheI-SwaI-EcoRI, about 350 bases of the 5' terminal portion of URA3 gene were amplified from plasmid pUTU-delsal and cloned using the primers (ura-sph-5, ura-sal-3) as shown under SEQ ID No:10 and 11. To NheI-SwaI site of this vector, about 460 bases of the 3' terminal portion of URA3 gene were subjected to PCR amplification and cloned using the primers (ura-nhe-5, ura-swa-3) as shown under SEQ ID No:12 and 13. In such manner, a plasmid containing DNA-1 for URA3 disruption was produced.

Next, to SalI-XhoI site of a plasmid containing DNA-1 for URA3 disruption, full-length of ADE1 gene of pUTA-1 was subjected to PCR amplification and inserted using the primers (ade-sal-5, ade-xho-3) as shown under SEQ ID No:14 and 15, and a plasmid containing DNA-2 for URA3 disruption was produced.

A plasmid containing DNA-3 for URA3 disruption (marker recovery type) was completed by subjecting about 630 bases of the 5' terminal portion of ADE1 gene to PCR amplification and cloning to XhoI-NheI site of a plasmid containing DNA-2 for URA3 disruption using the primers (ade-xho-5, ade-nhe-3) as shown under SEQ ID No:16 and 17.

A plasmid containing DNA for HIS5 disruption (marker recovery type) was produced by successively cloning about 500 bases of the 5' terminal portion of HIS5 gene cloned to pUC119 using the primers (his-sph-5, his-sal-3) as shown under SEQ ID No:18 and 19, and about 560 bases of the 3' terminal portion of HIS5 gene using the primer primers (his-nhe-5, his-swa-3) as shown under SEQ ID No:20 and 21 under the condition that SphI-SalI site and NheI-SwaI site of the plasmid containing DNA-3 for URA3 disruption being replaced.

### (Example 2) Disruption of URA3 gene of AC16 strain

The AC16 strain was cultured in YPD medium with a 10 ml large test tube overnight. This precultured yeast was inoculated on YM medium so as to be 1 ml/100 ml in a Sakaguchi flask, cultured for 6 hours, and cells were collected. The cells were suspended in 20 ml of 1 M sorbitol, and washed 3 times. Finally, the cells were suspended in 0.5 ml of 1 M sorbitol and used as competent cells. To 0.1 ml of the competent cells, 0.1 mg of DNA prepared by subjecting the plasmid containing DNA-2 for URA3 disruption of Example 1 to the restriction enzyme treatment with SphI and SwaI, and gene introduction by an electric pulse method was carried out. After applying electric pulse, 1 ml of 1 M sorbitol was put into a cuvet, the resultant was left under ice for 1 hour, and sown on SD plate. From the colony appeared, chromosomal DNA was extracted using a chromosome extraction kit Gentle-kun (product of TAKARA SHUZO CO., LTD.). Each 5 µg of the obtained chromosomal DNA was cut by three restriction enzyme treatments using ScaI, EcoT14I, and ScaI+EcoT14I, and subjected to electrophoresis in 0.8% agalose gel. According to Molecular cloning: A Laboratory Manual, Second Edition 9.31-9.57, Cold Spring Harbor Laboratory Press (1989), the resultants were transferred from gel to hybond N+ filter (product of Amersham Biosciences K.K.) one night. As a probe for southern blot detection, one prepared by subjecting an ScaI-NdeI fragment (340 bp), which is a sequence in URA3 gene, to enzyme-labeling using a gene image labeling/detection kit (product of Amersham Biosciences K.K.) was used. After hybridization, DNA was washed and DNA bands were detected with a fluorescence coloring reagent of the same kit. The detected band was compared with that of wild strain IAM12247, and a strain was selected which contains DNA treated with ScaI+EcoT14I and shows a band of about 570 bp in a wild strain newly showed a band of 1840 bp showing insertion of ADE1 gene into URA3 gene. This strain also showed a theoretical value in a band of a gene treated with ScaI or EcoT14I, and it was confirmed that one of URA3 genes was correctly disrupted by ADE1 gene.

Next, to competent cells prepared from this strain in the same manner as mentioned above, 0.5 mg of DNA prepared by amplifying a plasmid containing DNA-2 for URA3 disruption using the primer as shown under SEQ ID No:10 and 13 with the PCR method was added, and gene introduction was carried out in the same manner as mentioned above by the electric pulse method. After applying pulse, the cells were inoculated to 100 ml of YM medium, and cultured overnight. The cells were collected and cultured overnight in M medium (containing no ammonium sulfate). After the collection, the cells were transferred to M medium (containing ammonium sulfate), and cultured for 6 hours. Then, nystatin was added so as to be the final concentration of 0.01 mg/ml, and further cultured for 1 hour. The cells were washed and spread on an adenine-containing SD plate. From the red colony appeared, genomic DNA was extracted. The genomic DNA thus obtained of an adenine-requiring strain was amplified using the primers ura3-5 and ura3-3 shown under SEQ ID No:22 and 23. Then, DNA of 2.3 kbp amplified together with 0.9 kbp of URA3 gene which is intact in the original strain disappeared, and instead, DNA of 0.7 kbp was amplified. When the amplification was carried out using the primers adeY110-5 and adeY1670-3 shown under SEQ ID No:24 and 25, only DNA of 1.0 kbp was amplified. From these facts, it was determined that the entire region of ADE1 gene used for site-specifically disrupting the first URA3 gene was specifically removed. One of this adenine-requiring strain was named U-1 strain, and used for the following experiments.

Next, competent cells were prepared using a clone obtained using the U-1 strain. To 0.1 ml of the competent cells, 0.04 mg of DNA prepared by treating a plasmid containing DNA-3 for URA3 disruption with restriction enzymes SphI and SwaI and purifying was added, and gene introduction was carried out by the electric pulse method. The cells were spread on a uridine-containing SD plate, and incubated at 30°c. The appeared colony was replicated on SD plate and a uridine-containing SD plate, and a uracil-requiring strain was selected. Chromosomal DNA was collected therefrom and subjected to PCR amplification using the primers ura3-5 and ura3-3 shown under SEQ ID No:22 and 23. Then, a band of 0.9 kbp of intact URA3 gene was disappeared, and instead, 2.9 kbp, which is a size of DNA-3 for URA3 disruption, was amplified. The amplification was carried out using the primers adeY110-5 and adeY1670-3 shown under SEQ ID No:24 and 25, and then ADE1 gene of 1.5 kbp which is not amplified in the original strain was amplified. From these facts, it was determined that the gene 3 for disruption was introduced specifically for URA3 gene, whole of URA3 gene was disrupted, and uracil requirement was acquired. Then, nutritional requirement of the obtained strain was confirmed to be complemented with vector pUTU-2. This uracil-requiring strain was named Candida maltosa U-35.

Next, Candida maltosa U-35 strain was cultured in 10 ml of YPD medium overnight. After the collection, the strain was cultured in M medium (containing no ammonium sulfate) one night. The cells were collected, transferred to uridine-containing M medium (containing ammonium sulfate), cultured for 7 hours, added with nystatin so as to be the final concentration of 0.01 mg/ml, and further cultured for 1 hour. The cells were washed, and then cultured in adenine- and uridine-containing M medium (containing ammonium sulfate) overnight. The cells were collected, cultured in M medium (containing no ammonium sulfate) overnight, transferred to uridine-containing M medium (containing ammonium sulfate) for 7 hours, subjected to nystatin treatment as mentioned previously, and spread on an adenine- and uridine-containing SD plate. After the culture, the obtained red colony was replicated on an SD plate, uridine-containing SD plate, and adenine- and uridine-containing SD plate, and was confirmed to be a clone showing nutritional requirement of both adenine and uracil. Genomic DNA was extracted from this strain, and subjected to PCR amplification using the primers ura3-5 and ura3-3 shown under SEQ ID No:22 and 23. Then, a band of 2.9 kbp which is a band of URA3 gene introduced with ADE1 gene disappeared, and instead, 1.2 kbp, which is a size of an ADE1 gene fragment remained in URA3 gene, was amplified. The amplification was carried out using the primers adeY110-5 and adeY1670-3 shown under SEQ ID No:24 and 25, and then in the parental strain, ADE1 gene of 1.5 kbp was amplified, but in the obtained nutrition-requiring strain, only a band of 1.0 kbp, which is the original size of ADE1-disrupted gene was confirmed. From these facts, it was shown that ADE1 gene and an ADE1 gene fragment in URA3 gene were deleted spontaneously by the homologous recombination within the same gene, and that a gene marker could be recovered with ease. This double nutrition-requiring strain, i.e. adenine and uracil, was named Candida maltosa UA-354.

### (Example 3) HIS5 gene disruption in AC16 strain and method for recovering a marker

Competent cells were prepared from the U-1 strain produced in Example 2, added with 0.04 mg of DNA prepared by treating a plasmid containing DNA for HIS5 disruption with restriction enzymes SphI and SwaI and purifying the resultant, and then subjected to gene introduction by the electric pulse method. The conditions were the same as in Example 2. These cells were spread on a histidine-containing SD plate, and incubated at 30°c. Genomic DNA was collected from the appeared colony. Amplification of genomic DNA was carried out using the primers of flanking site of the portion having homology with HIS5 gene of the gene for disruption in HIS5 gene, that is his-sal2 and his-1900 (SEQ ID No:26 and 27), which are primers of HIS5 gene not contained in the gene for disruption. Then, a strain in which a band of 1.9 kbp, which is a size of intact HIS5 gene, and a band of 3.4 kbp, which is a size of DNA for HIS5 disruption, were amplified was selected.

Using this strain, by the same method as shown in Example 2, nystatin concentration was carried out. The strain was spread on an adenine-containing SD plate, genome DNA was extracted from the obtained red colony, and the amplification of genome DNA was carried out using the primers his-sa12 and his-1900 (SEQ ID No:26 and 27). Then, amplification of only a band of 1.9 kbp, which is a size of intact HIS5 gene, was confirmed, but a band of 3.4 kbp, which is a size containing the gene for disruption was not amplified. PCR amplification was carried out using the primers ade-xho-5 and his-swa-3 (SEQ ID No:16 and 21), and then a band of 1.2 kbp which is a band of an ADE1 gene fragment coupled to HISS gene was amplified. Thus, it was confirmed that the obtained adenine-requiring strain was not a revertant, and was obtained as a result of the intramolecular homologous recombination.

Next, competent cells were prepared from the obtained adenine-requiring strain, added with 0.05 mg of DNA prepared by treating a plasmid containing DNA for HIS5 disruption with restriction enzymes SphI and SwaI and purifying the resultant, and then the resultant was subjected to gene introduction by the electric pulse method. The cells were spread on a histidine-containing SD plate, and incubated at 30°c. The obtained colony was replicated to an SD plate and histidine-containing SD plate to obtain a histidine-requiring strain. Genome DNA was extracted from the obtained histidine-requiring strain, and amplification of genome DNA was carried out using the primers his-sal2 and his-1900 (SEQ ID No:26 and 27). Then, a strain in which a band of 3.4 kbp, which is a size containing a gene for disruption, other than a band of 1.9 kbp, which is a size of disrupted HIS5 gene being amplified in the parental strain, was selected. This strain was confirmed to be incorporated with ADE1 gene into HIS5 gene by PCR using the primers his-sal2 and ade-xho-3 (SEQ ID No:26 and 15). This histidine-requiring strain was named Candida maltosa CH-I strain.

Using the CH-I strain, nystatin concentration was carried out by the same method as shown in Example 2. The strain was spread on an adenine- and histidine-containing SD plate, and genome DNA was extracted from the obtained red colony. Amplification of genome DNA was carried out using the primers his-sal2 and his-1900 (SEQ ID No:26 and 27). Then, in all the strains, only a band of 1.9 kbp, which is a size of HIS5 gene disrupted with the parental strain, was confirmed, and a band of 3.4 kbp, which is a size of containing a gene for disruption, was not amplified. Histidine and adenine requirement were confirmed by replications to an SD plate, histidine-containing SD plate, and adenine- and histidine-containing SD plate, and thus it was considered as completion of double nutrition-requiring strain, i.e. adenine and histidine. One of the strains was named Candida maltosa AH-15 strain.

Next, competent cells were prepared from the AH-15 strain, added with 0.025 mg of DNA prepared by treating a plasmid containing DNA-3 for URA3 disruption with restriction enzymes SphI and SwaI and purifying the resultant, and then the resultant was subjected to gene introduction by the electric pulse method. The cells were spread on a uridine- and histidine-containing SD plate, and incubated at 30°c for two days. The appeared colony was replicated to a histidine-containing SD plate and uridine-and histidine-containing SD plate, and a uracil-requiring strain was selected. From this strain, chromosomal DNA was collected, and PCR amplification was carried out using the primers ura3-5 and ura3-3 (SEQ ID No:22 and 23). Then, a band of 0.9 kbp of intact URA3 gene disappeared, and instead, amplification of 2.9 kbp, which is a size of a gene for disruption was confirmed. One of the double nutrition-requiring strain, i.e. histidine and uracil, was named Candida maltosa HU-591.

Using the HU-591 strain, in the same method as shown in Example 2, nystatin concentration was carried out. The cells were spread on an adenine-, histidine-, and uridine-containing SD plate, and genome DNA was extracted from the obtained red colony. PCR amplification was carried out using the primers ura3-5 and ura3-3 (SEQ ID No:22 and 23), and a strain in which a band of 2.9 kbp which is a size of ADE1 gene being introduced into URA3 gene disappeared, and instead, a band of 1.2 kbp, which is a size of URA3 gene with ADE1 gene fragment being remained, was selected. Uracil, histidine, and adenine requirement was confirmed by carrying out replication to an adenine-, histidine- and uridine-containing SD plate, a histidine- and uridine-containing SD plate, an adenine and uridine-containing SD plate, and adenine- and histidine-containing SD plate, an adenine-containing SD plate, a histidine-containing SD plate, a uridine-containing SD plate, and to an SD plate. Thus, a triple nutrition-requiring strain, i.e. adenine, histidine and uracil was completed. This strain was named Candida maltosa AHU-71.

The appearance frequency of an adenine-requiring strain in the case where a marker recovery type gene for disruption was used was equal to the case where DNA for adenine disruption was used, but the time required until the acquisition of the target strain could be shortened to approximately half. Furthermore, it was not necessary to take insertion of a gene for disruption into the site other than targeted at the time of introduction into consideration, and the analysis was also easy.

As shown in this Example, it was shown that a marker gene can be easily recovered using the intramolecular homologous recombination.

### (Example 4) Confirmation of fats and oils assimilation ability

Using the AHU-71 strain, which is a finally completed triple nutrition-requiring strain, jar culture was carried out in order to confirm that there is no problem in the growth when using fats and oils as the carbon source. To the AHU-71 strain, plasmid pUTU2-Ade-His was transformed, and a colony was caused to form in an SD plate. As a control, one prepared by transforming plasmid pUTA-1 to AC16 strain was used. A mother stock was cultured and prepared in a Sakaguchi flask using 150 ml of SD medium. Jar culture was carried out by charging 1.8 L of M2 medium to a 3 L jar fermenter manufactured by B. E. Marubishi Co., Ltd. The condition were set as follows: temperature 32°c, the stirring rate 500 rpm, and the ventilation amount 1 vvm. As the carbon source, palm kernel oil was used, and fed at 1.9 ml/h from the start of culture to the 11th hour, at 3.8 ml/h until the 24th hour, and at 5.7 ml/h for the rest of the time. 10 ml of the culture fluid was sampled with time, washed with methanol, and dried to determine the dried cell weight. As shown in Fig. 2, the AHU-71 strain showed the similar growth as that of AC16 strain. Thereby, it was confirmed that gene disruption has been performed in this strain without impairing fats and oils assimilation ability.

### (Example 5) Construction of an enzyme gene expression cassette involved with polyester synthesis

For expressing a polyester synthase in Candida maltosa, a promoter derived from Candida maltosa was jointed to the 5' upstream, and a terminator was jointed to the 3' downstream. As the promoter, promoter ARRp in which ARR sequence was added to the upstream of a promoter of ALK2 gene (GenBank: X55881) was jointed. To the 3' downstream, terminator ALK1t of ALK1 gene of Candida maltosa (GenBank: D00481) was jointed. ARRp was converted to the form which can be cut with XhoI and NdeI by coupling EcoRI-XhoI linker to PstI site of the gene imparted from Tokyo University (SEQ ID No:4) and coupling synthesized DNA shown under SEQ ID No:28 to EcoT14I site. After cutting pUAL1 (WO 01/88144) with EcoRI, by carrying out blunting and ligation, pUAL2 removed with EcoRI cutting site was produced. pUAL2 was cut with PuvII/PuvI, and coupled to SmaI/PuvII site of pSTV28 (product of TAKARA SHUZO CO., LTD.) to produce pSTAL1. This pSTAL1 was cut with EcoRI/NdeI, and coupled with ARRp mentioned before to produce pSTARR.

The peroxisome-targeting signal was added to the carboxy terminal so that phaCac149NS shown under SEQ ID No:2 targets to peroxisome. As the added peroxisome-targeting signal, an amino acid of Ser-Lys-Leu (SKL) was used to the carboxy terminal. Next, by using phaCac149NS cloned in pUCNT as a template, gene amplification was carried out using the primers shown under SEQ ID No:29 and 30, and the resultant was coupled to NdeI and PstI site of pSTARR to construct pSTARR-phaCac149NS. The primers shown under SEQ ID No:31 to 35 were used to confirm the base sequence. For the base sequence determination, DNA sequencer 310 Genetic Analyzer manufactured by PERIKIN ELMER APPLIED BIOSYSTEMS, Inc. was used.

Next, pSTARR-phbB was constructed by adding the peroxisome-targeting signal by amplification with the primers shown under SEQ ID No:36 and 37 to a carboxy terminal of a chemically-synthesized acetoacetyl CoA reductase gene (phbB) derived from Ralstonia eutropha (Ralstonia eutropha, H16 strain, ATCC17699) shown under SEQ ID No:3 in which a codon was converted for Candida maltosa, and coupled to NdeI and PstI sites of the above pSTARR. The base sequence was confirmed by the same method as mentioned above.

To SalI site of pUTA-1 which is a vector for Candida maltosa, two synthase expression cassettes cut from pSTARR-phaCac149NS with SalI and XhoI were introduced, and pARR-149NSx2 was produced. Furthermore, to SalI site of this vector, one phbB expression cassette cut from pSTARR-phbB with SalI and XhoI was introduced, and pARR-149NSx2-phbB was produced.

DNA for introduction for introducing a heterogene into disrupted HIS5 gene site on the chromosome of Candida maltosa was produced using DNA for HIS5 disruption described in Example 1. DNA for HIS5 disruption was cut with SalI and XhoI, ADE1 gene was removed, and a plasmid introduced with URA3 gene cut from pUTU-delsal with SalI and XhoI was produced. An expression cassette was cut from the above pSTARR-phaCac149NS with SalI and XhoI, and coupled with SalI site of this vector. Then, a plasmid containing DNA-1 for introduction was produced by cutting an expression cassette from pSTARR-phaCac149NS with SalI and XhoI and coupling thereof to XhoI site of the above-mentioned plasmid.

Furthermore, a plasmid containing DNA-2 for introduction was produced by coupling a phbB expression cassette cut from pSTARR-phbB with SalI and XhoI to XhoI site of the plasmid containing DNA-1 for introduction.

DNA for introduction for introducing a heterogene into disrupted URA3 gene site on the chromosome of Candida maltosa was produced using DNA-1 for URA3 disruption described in Example 1. A plasmid in which HIS5 gene amplified by PCR with the primers shown under SEQ ID No:38 and 39 was introduced into SalI-XhoI site of the plasmid containing DNA-1 for URA3 disruption was produced. To SalI site of this plasmid, an expression cassette cut from the above pSTARR-phaCac149NS with SalI and XhoI was coupled. Then, a plasmid containing DNA-3 for introduction was produced by cutting an expression cassette from pSTARR-phbB with SalI and XhoI and coupling this to XhoI site of this vector. A plasmid containing DNA-4 for introduction was also produced by coupling the expression cassette of phbB in lieu of phaC expression cassette to SalI site.

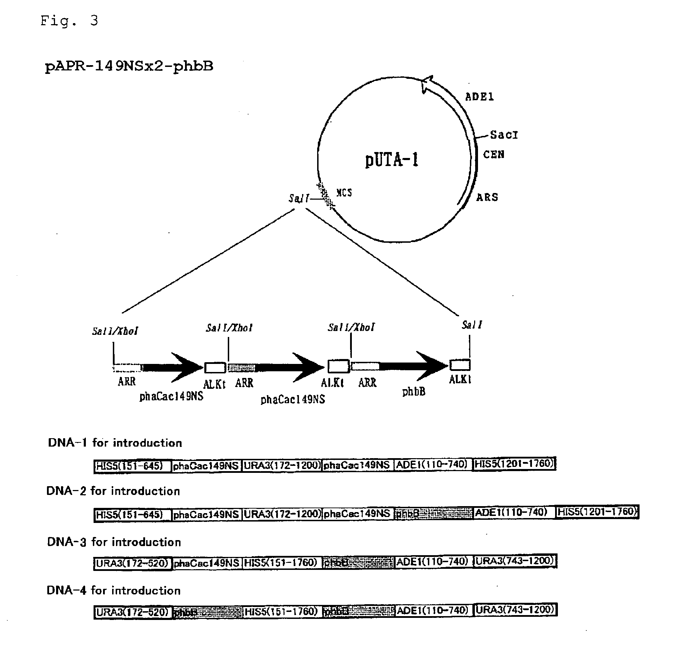

In Fig. 3, schematic illustrations of the produced DNA-1 to 4 for gene introduction were shown. In Fig. 3, the numbers in parentheses represent the number from the 5' terminal of the genes registered on GenBank of the respective gene fragments used. ADE1 gene: D00855, URA3 gene: D12720, and HIS5 gene: X17310. The sites surrounded by a heavy line represent the homology sites with chromosomal DNA.

### (Example 6) Construction of a recombinant

Using the Candida maltosa AHU-71 strain produced in Example 3, competent cells for electroporation were prepared by the method described in Example 2. To the competent cells, 0.05 mg of DNA-1 and 2 for introduction treated with restriction enzymes NotI and SwaI were electroporated, and spread on an adenine- and histidine-containing SD plate. From the colony appeared, chromosomal DNA was prepared and PCR was carried out using the primers shown under SEQ ID No:26 and 27. A colony in which genes equivalent the size of DNA-1 and 2 for introduction were amplified besides the gene of 1.9 kbp equivalent to the disrupted HIS5 gene was selected as a strain introduced into the HIS5 gene site. Furthermore, by PCR using various primers, it was confirmed that there was no deletion in these introduced genes, etc.

Next, from these strains, the competent cells for electroporation were prepared in the same manner. To the competent cells, 0.05 mg of DNA-3 and 4 for introduction treated with restriction enzymes NotI and SwaI were electroporated and spread on an adenine-containing SD plate. From the appeared colony, chromosomal DNA was prepared and PCR was carried out using the primers shown under SEQ ID No:22 and 23. A colony in which the amplification of either 0.7 kbp gene equivalent to disrupted URA3 gene or 1.2 kbp gene was not confirmed, and instead, genes equivalent to the sizes of DNA-3 and 4 for introduction were amplified was selected as a strain introduced into the URA3 gene site. Furthermore, by PCR using various primers, it was confirmed that there was no deletion in these introduced genes, etc.

pARR-149NSx2-phbB produced in Example 5 was transformed to the Candida maltosa AC 16 strain, and the strain in which 2 copies of phaCac149NS expression cassette and 1 copy of phbB expression cassette were introduced was named A strain. The strain in which 2 copies of phaCac149NS expression cassette and 2 copies of phbB expression cassette were inserted into a chromosome of the Candida maltosa AHU-71 strain, produced using DNA-1 and 4 for introduction, was named B strain. The strain in which 3 copies of phaCac149NS expression cassette and 1 copy of phbB expression cassette were inserted into a chromosome of the Candida maltosa AHU-71 strain, produced using DNA-1 and 3 for introduction, was named C strain. The strain in which 3 copies of phaCac149NS expression cassette and 2 copies of phbB expression cassette were inserted into a chromosome of Candida maltosa AHU-71 strain, produced using DNA-2 and 3 for introduction, was named D strain. pARR-149NSx2-phbB was transformed to C strain, and E strain in which 5 copies of phaCac149NS expression cassette and 2 copies of phbB expression cassette were introduced was produced. pARR-149NSx2-phbB was transformed to D strain, and F strain in which 5 copies of phaCac149NS expression cassette and 3 copies of phbB expression cassette were introduced was produced. pARR-149NSx2-phbB was transformed to a strain in which 2 copies of phaCac149NS expression cassette and 3 copies of phbB expression cassette were inserted into a chromosome produced using DNA-2 and 4 for introduction, and G strain in which 4 copies of phaCac149NS expression cassette and 4 copies of phbB expression cassette were introduced was produced. This G strain was named CM313-X2B, and internationally deposited (FERM BP-08622). Similarly, as a control, a strain in which pUTA-1 was transformed to the Candida maltosa AC16 strain (control-1), and a strain in which pARR-149NSx2 was transformed to Candida maltosa AC16 strain (control-2) were also produced. The brief summary of the produced strains was shown in Table 1.

**Table 1**

| | Host | Plasmid | Chromosome introduction | | Total number of expression cassette | |
|---|---|---|---|---|---|---|
| | | | HIS5 locus | URA3 locus | phaC | phbB |
| control-1 | AC16 | pUTA-1 | None | None | 0 | 0 |
| control-2 | AC16 | pARR-149NS_{×}2 | None | None | 2 | 0 |
| A | AC16 | pARR-149NSₓ2-phbB | None | None | 2 | 1 |
| B | AHU-71 | pUTA-1 | DNA-1 for introduction | DNA-4 for introduction | 2 | 2 |
| C | AHU-71 | pUTA-1 | DNA-1 for introduction | DNA-3 for introduction | 3 | 1 |
| D | AHU-71 | pUTA-1 | DNA-2 for introduction | DNA-3 for introduction | 3 | 2 |
| E | AHU-71 | pARR-149NS_{×}2-phbB | DNA-1 for introduction | DNA-3 for introduction | 5 | 2 |
| F | AHU-71 | pARR-149NS_{×}2-phbB | DNA-2 for introduction | DNA-3 for introduction | 5 | 3 |
| G | AHU-71 | pARR-149NS_{×}2-phbB | DNA-2 for introduction | DNA-4 for introduction | 4 | 4 |

### (Example 7) Polymer production using a recombinant

A Candida maltosa recombinant introduced with the gene necessary for the polymer production was cultured as follows. SD medium was used for preculture, and M2 medium containing palm kernel oil as the carbon source was used as a production medium. 500 µl of glycerol stock of each recombinant was inoculated into a 500 ml Sakaguchi flask containing 50 ml of the preculture medium, cultured for 20 hours, and then inoculated into a 2 L Sakaguchi flask containing 300 ml of the production medium at an inoculum size of 10 v/v %. This was cultured at the culture temperature of 30°c, and shaking speed of 90 rpm for 2 days. From the culture fluid, cells were collected by centrifugation, suspended in 80 ml of distilled water, and disrupted in an ultrahigh pressure homogenizer (APV's Rannie 2000, at 15,000 Psi for 15 minutes), followed by centrifugation. The precipitate obtained was washed with methanol and then lyophilized. The lyophilized cells were ground, and 1 g was weighed. To this was added with 100 ml of chloroform was added, and the mixture was stirred overnight and extracted. The cells were removed by filtration, the filtrate was concentrated to 10 ml using an evaporator, and about 50 ml of hexane was added to the concentrate to precipitate and dry the polymer. The composition of the obtained polymer was analyzed by NMR analysis (JEOL Ltd., JNM-EX400). The weight average molecular weight was measured as follows. The harvested dried polymer (10 mg) was dissolved in 5 ml of chloroform, and the obtained solution was analyzed by Shimadzu Corporation's GPC system equipped with Shodex K805L (300 x 8 mm, two columns were connected)(product of Showa Denko K. K.) using chloroform as a mobile phase. As the standard molecular weight sample, commercial standard polystyrene was used. The results were shown in Table 2.

**Table 2**

| | Polymer content (weight%) | 3HH fraction (mol%) | Weight average molecular weight (M.W.) |
|---|---|---|---|
| control-1 | 0 | - | - |
| control-2 | 14 | - | - |
| A | 25 | 18.1 | 800000 |
| B | 35 | 12.4 | 1200000 |
| C | 31 | 18.9 | 600000 |
| D | 40 | 14.3 | 1000000 |
| E | 43 | 15.5 | 350000 |
| F | 45 | 14.6 | 550000 |
| G | 45 | 12.2 | 900000 |

By the above results, it was made clear that a PHA can be produced quite efficiently by using the novel gene-disrupted yeast and the novel mutant of the present invention. Moreover, it became clear that it is very important for the efficient polyester production to introduce one of the genes involved with polyester biosynthesis in 2 or more copies, and the molecular weight and composition can be controlled by the introduction number of the expression cassette.

### INDUSTRIAL APPLICABILITY

The yeast having a plurality of markers produced by gene disruption of the present invention is expectable to be used for highly efficient gene expression or gene expression product production as a host for gene recombination. Moreover, by the present invention, it becomes possible to efficiently produce a copolyester producible by copolymerizing 3-hydroxyalkanoic acid having biodegradability and excellent physical properties within yeast. In addition, it also becomes possible to add a marker made by gene disruption, which leads to development of a better host.

By the present invention, it becomes possible to efficiently produce a copolyester producible by copolymerizing 3-hydroxyalkanoic acid having biodegradability and excellent physical properties represented by the above general formula (1) within yeast. Further, control of the physical properties of copolyesters becomes possible. Furthermore, it also becomes possible to efficiently carry out gene introduction in yeast more than once.

## Claims

1. A uracil-requiring gene-disrupted yeast
wherein URA3 gene of chromosomal DNA is disrupted by the homologous recombination with a URA3 DNA fragment.

2. A histidine-requiring gene-disrupted yeast
wherein HIS5 gene of chromosomal DNA is disrupted by the homologous recombination with an HIS5 DNA fragment.

3. An adenine- and uracil-requiring gene-disrupted yeast
wherein ADE1 gene and URA3 gene of chromosomal DNA are disrupted by the homologous recombination with an ADE1 DNA fragment and URA3 DNA fragment.

4. An adenine- and histidine-requiring gene-disrupted yeast
wherein ADE1 gene and HIS5 gene of chromosomal DNA are disrupted by the homologous recombination with an ADE1 DNA fragment and HIS5 DNA fragment.

5. A uracil- and histidine-requiring gene-disrupted yeast
wherein URA3 gene and HIS5 gene of chromosomal DNA are disrupted by the homologous recombination with a URA3 DNA fragment and HIS5 DNA fragment.

6. An adenine-, uracil- and histidine-requiring gene-disrupted yeast
wherein ADE1 gene, URA3 gene and HIS5 gene of chromosomal DNA are disrupted by the homologous recombination with an ADE1 DNA fragment, URA3 DNA fragment and HIS5 DNA fragment.

7. The gene-disrupted yeast according to any one of Claims 1 to 6,
wherein the yeast is one belonging to the genus Candida, the genus Clavispora, the genus Cryptococcus, the genus Debaryomyces, the genus Lodderomyces, the genus Metschnikowia, the genus Pichia, the genus Rhodosporidium, the genus Rhodotorula, the genus Sporidiobolus, the genus Stephanoascus, or the genus Yarrowia.

8. The gene-disrupted yeast according to any one of Claims 1 to 6,
wherein the yeast belongs to the genus Candida.

9. The gene-disrupted yeast according to any one of Claims 1 to 6,
wherein the yeast is the albicans species, ancudensis species, atmosphaerica species, azyma species, bertae species, blankii species, butyri species, conglobata species, dendronema species, ergastensis species, fluviatilis species, friedrichii species, gropengiesseri species, haemulonii species, incommunis species, insectrum species, laureliae species, maltosa species, melibiosica species, membranifaciens species, mesenterica species, natalensis species, oregonensis species, palmioleophila species, parapsilosis species, pseudointermedia species, quercitrusa species, rhagii species, rugosa species, saitoana species, sake species, schatavii species, sequanensis species, shehatae species, sorbophila species, tropicalis species, valdiviana species, or viswanathii species of the genus Candida.

10. The gene-disrupted yeast according to any one of Claims 1 to 6,
wherein the yeast is Candida maltosa.

11. The URA3 gene-disrupted yeast according to Claim 1
which is Candida maltosa U-35 (FERM P-19435).

12. The HIS5 gene-disrupted yeast according to Claim 2
which is Candida maltosa CH-I (FERM P-19434).

13. The ADE1 gene- and URA3 gene-disrupted yeast according to Claim 3
which is Candida maltosa UA-354 (FERM P-19436).

14. The ADE1 gene- and HIS5 gene-disrupted yeast according to Claim 4
which is Candida maltosa AH-15 (FERM P-19433).

15. The URA3 and HIS5 gene-disrupted yeast according to Claim 5
which is Candida maltosa HU-591 (FERM P-19545).

16. The ADE1 gene-, URA3 gene- and HIS5 gene-disrupted yeast according to Claim 6,
which is Candida maltosa AHU-71 (FERM BP-10205).

17. A transformant of the gene-disrupted yeast according to any one of Claims 1 to 16,
which is transformed with a DNA sequence containing an isogene or heterogene.

18. A process for producing a gene expression product
which comprises harvesting an expression product of an isogene or heterogene from a cultured product obtainable by culturing the transformant according to Claim 17.

19. The process for producing a gene expression product according to Claim 18,
wherein the gene expression product is a polyester.

20. A yeast transformant
which is introduced with a polyhydroxyalkanoic acid synthase gene and an acetoacetyl CoA reductase gene, and both or either of these genes being introduced in 2 or more copies.

21. The yeast transformant according to Claim 20,
wherein a peroxisome-targeting signal is added to a polyhydroxyalkanoic acid synthase gene and an acetoacetyl CoA reductase gene.

22. The yeast transformant according to Claim 20 or 21,
wherein a promoter and terminator functioning in yeast are connected to a polyhydroxyalkanoic acid synthase gene and acetoacetyl CoA reductase gene.

23. The yeast transformant according to any one of Claims 20 to 22,
wherein the yeast belongs to the genus Candida.

24. The yeast transformant according to any one of Claims 20 to 22,
wherein the yeast is the albicans species, ancudensis species, atmosphaerica species, azyma species, bertae species, blankii species, butyri species, conglobata species, dendronema species, ergastensis species, fluviatilis species, friedrichii species, gropengiesseri species, haemulonii species, incommunis species, insectrum species, laureliae species, maltosa species, melibiosica species, membranifaciens species, mesenterica species, natalensis species, oregonensis species, palmioleophila species, parapsilosis species, pseudointermedia species, quercitrusa species, rhagii species, rugosa species, saitoana species, sake species, schatavii species, sequanensis species, shehatae species, sorbophila species, tropicalis species, valdiviana species, or viswanathii species of the genus Candida.

25. The yeast transformant according to any one of Claims 20 to 22,
wherein the yeast is Candida maltosa.

26. The yeast transformant according to any one of Claims 20 to 25,
wherein the polyhydroxyalkanoic acid synthase gene codes for an enzyme or mutant derived from Aeromonas caviae having the amino acid sequence shown under SEQ ID No:5.

27. The yeast transformant according to Claim 26,
wherein the polyhydroxyalkanoic acid synthase gene derived from Aeromonas caviae codes for a polyhydroxyalkanoic acid synthase mutant obtainable by applying at least one of the following amino acid substitutions from (a) to (h);
(a) substitution of Ser for Asn-149
(b) substitution of Gly for Asp-171
(c) substitution of Ser or Gln for Phe-246
(d) substitution of Ala for Tyr-318
(e) substitution of Ser, Ala or Val for Ile-320
(f) substitution of Val for Leu-350
(g) substitution of Thr, Ser or His for Phe-353
(h) substitution of Ile for Phe-518.

28. The yeast transformant according to any one of Claims 20 to 27,
wherein the acetoacetyl CoA reductase gene codes for an enzyme or mutant derived from Ralstonia eutropha having the amino acid sequence shown under SEQ ID NO:6.

29. The yeast transformant according to any one of Claims 20 to 28,
wherein the polyhydroxyalkanoic acid is a copolymer obtainable by copolymerizing 3-hydroxyalkanoic acid represented by the following general formula (1); 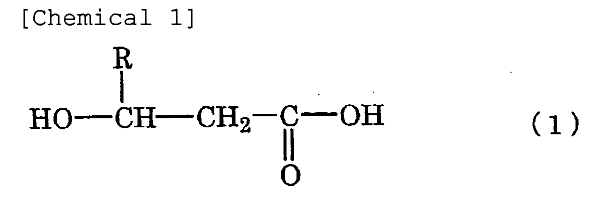 in the formula, R represents an alkyl group having 1 to 13 carbon atoms.

30. The yeast transformant according to any one of Claims 20 to 28,
wherein the polyhydroxyalkanoic acid is a copolyester obtainable by copolymerizing 3-hydroxybutyric acid represented by the following general formula (2) and 3-hydroxyhexanoic acid represented by the following general formula (3).

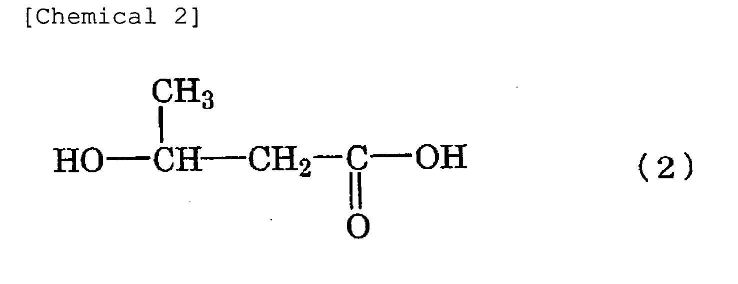

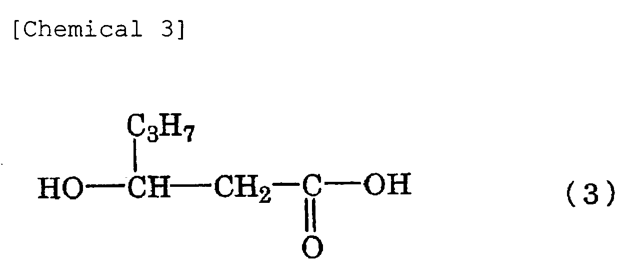

31. A process for producing a polyester using the yeast transformant according to any one of Claims 20 to 30,
which comprises harvesting a polyester from a cultured product obtainable by culturing said yeast transformant.

32. A method for controlling the molecular weight of a polyester in producing a polyester using the yeast transformant according to any one of Claims 20 to 30,
which comprises controlling the number of acetoacetyl CoA reductase gene in the yeast transformant.

33. A method for controlling a hydroxyalkanoic acid composition of a polyester in producing a polyester using the yeast transformant according to any one of Claims 20 to 30,
which comprises controlling the number of a polyhydroxyalkanoic acid synthase gene in the yeast transformant.

34. A method for recovering a selective marker
which comprises carrying out the intramolecular homologous recombination in Candida maltosa having ADE1 gene as a selective marker gene to remove said ADE1 gene.

35. The method for recovering a selective marker according to Claim 34,
wherein a part of ADE1 gene is ligated to the upstream or downstream of ADE1 gene.

36. The method for recovering a selective marker according to Claim 34 or 35,
wherein ADE1 gene has the base sequence shown under SEQ ID NO:7.
